# EUROPÄISCHE PATENTSCHRIFT

(11) **EP 2 571 431 B1**
(45) Veröffentlichungstag und Bekanntmachung des Hinweises auf die Patenterteilung: **25.06.2014**
(21) Anmeldenummer: 11722736.3
(22) Anmeldetag: 18.05.2011
(51) Int. Cl.: A61B 17/3207

(54) **MEDIZINISCHE VORRICHTUNG ZUM ENTFERNEN VON KONKREMENTEN**
MEDICAL DEVICE FOR REMOVING CONCRETIONS
DISPOSITIF MÉDICAL POUR ÉLIMINER DES CALCULS

(30) Priorität: 18.05.2010 DE 102010020869; 14.09.2010 DE 102010045367
(43) Veröffentlichungstag der Anmeldung: 27.03.2013
(73) Patentinhaber: Acandis GmbH & Co. KG, 76327 Pfinztal (DE)
(72) Erfinder: NAGL, Frank, 76137 Karlsruhe (DE); MAILÄNDER, Werner, 75331 Engelsbrand Grunbach (DE); CATTANEO, Giorgio, 76199 Karlsruhe (DE); SCHÜSSLER, Kirsi, 75177 Pforzheim (DE)
(74) Vertreter: Kilchert, Jochen
(86) Internationale Anmeldenummer: PCT/EP2011/002483
(87) Internationale Veröffentlichungsnummer: WO 2011/144336

(56) Entgegenhaltungen:
- WO-A1-2009/154441
- WO-A2-2007/089897
- US-A- 5 527 326
- US-A- 5 904 698
- US-A1- 2010 036 410

## Beschreibung

Die Erfindung betrifft eine medizinische Vorrichtung, zum Entfernen von Konkrementen aus Körperhohlorganen gemäß dem Oberbegriff den Patentansprüche 1 und 16. Eine derartige Vorrichtung ist beispielsweise aus der WO 2006/031410 A2 und der WO 2007/089897 A2 bekannt.

Zur Behandlung von Thrombosen, insbesondere in der interventionellen Neuroradiologie, werden in der Praxis Thrombektomiesysteme eingesetzt. Dabei wird der die Thrombose auslösende Thrombus bzw. das Blutgerinnsel mechanisch aus dem Blutgefäß entfernt. Ein derartiges System ist in der eingangs genannten WO 2006/031410 A2 beschrieben.

Das bekannte System umfasst einen Katheter, der zur Zufuhr eines korbartigen Fangelements angepasst ist. Das korbartige Fangelement umfasst eine Gitterstruktur, die von einem komprimierten Zustand in einen expandierten Zustand überführbar ist. Im expandierten Zustand ist das Fangelement distal des Katheters angeordnet und weist eine rotationssymmetrische Struktur auf.

Bei dem bekannten System wird das Fangelement durch den Katheter an den Behandlungsort geführt. Dort wird das Fangelement freigesetzt und expandiert, so dass sich die korbartige Struktur des Fangelements ausbildet. Der Katheter ist ferner mit einer Absaugeinheit verbunden, so dass ein Unterdruck im Bereich des korbartigen Fangelements erzeugbar ist, der den Thrombus in das Fangelement zieht. Dabei liegt die Gitterstruktur des Fangelements an der Gefäßwand des zu behandelnden Blutgefäßes an. Der Thrombus wird in dem Fangelement eingekapselt und kann durch das Fangelement aus dem Blutgefäß gezogen werden, wobei die Gitterstruktur des Fangelements entlang der Gefäßwand des Blutgefäßes gleitet.

Durch den Kontakt des Fangelements mit der Gefäßwand des zu behandelnden Blutgefäßes wird eine mechanische Beanspruchung der Blutgefäßwand bewirkt. Dadurch können sich Verletzungen in der Gefäßwand ausbilden. Die Bewegung des Fangelements entlang der Gefäßwand verstärkt diesen Effekt. Im Allgemeinen weisen bekannte Systeme, insbesondere das System gemäß der eingangs genannten WO 2006/031410 A2, den Nachteil auf, die Gefäßwände des zu behandelnden Körperhohlorgans einer erhöhten Belastung auszusetzen.

Der Erfindung liegt die Aufgabe zu Grunde, eine medizinische Vorrichtung zum Entfernen von Konkrementen aus Körperhohlorganen anzugeben, bei der das Risiko von Komplikationen durch Verletzung von Gefäßwänden reduziert ist. Ferner soll eine medizinische Vorrichtung zum Entfernen von Konkrementen aus Körperhohlorganen angegeben werden, die eine verbesserte Handhabung und Anwendungssicherheit aufweist.

Erfindungsgemäß wird diese Aufgabe durch den Gegenstand des Patentanspruchs 1, alternativ durch den Gegenstand des nebengeordneten Patentanspruchs 16 gelöst.

Die Erfindung hat den Vorteil, dass durch die Kombination des Schneidbereichs mit dem Halteelement die Verankerung des Konkrements am Funktionselement auch unter schwierigen Bedingungen verbessert wird, beispielsweise wenn das Konkrement durch stark gekrümmte oder sich aufweitende Gefäßquerschnitte durch das Funktionselement transportiert wird. Das Halteelement folgt dabei dem Konkrement, selbst wenn der Schneidbereich zumindest zeitweise und/oder teilweise mit dem Konkrement außer Eingriff kommt. Dadurch wird die Haltefunktion aufrecht erhalten und das Konkrement bleibt am Funktionselement fixiert.

Bei einem bevorzugten Ausführungsbeispiel bildet das Halteelement eine zungenförmige Zelle mit einer freien Spitze, die in einer rautenförmigen Gitterzelle und/oder Masche der Gitterstruktur angeordnet ist.

Bei dem Ausführungsbeispiel handelt es sich um eine Zelle-in-Zelle Anordnung, bei der die Außenzelle, die das Halteelement umgibt, rautenförmig ist. Das Haltelement ist dabei mit der Außenzelle verbunden. Die Rautenform der Außenzelle verbessert die Flexibilität der Struktur. Das Funktionselement ist daher besonders gut dazu angepasst ist, im Konkrement zu expandieren und dieses von innen heraus zu halten. Zum Lösen und Entfernen des Konkrements wird eine in proximaler Richtung wirkende Zugkraft auf das expandierte Funktionselement ausgeübt. Diese führt auf Grund der Rautenform zu einer Streckung der Außenzelle. Durch die Streckung der Außenzelle wird das Halteelement bzw. die in der Außenzelle angeordnete zungenförmige Zelle gestreckt und verlängert sich. Die Verlängerung des Halteelements geht mit einer Streckung bzw. Auslenkung des Halteelements radial nach außen einher. Überdies wird das Halteelement bzw. die in der Außenzelle angeordnete zungenförmige Zelle beim Komprimieren versteift. Ein weitere Effekt der Streckung des Halteelements besteht darin, dass das Halteelement scherenartig geschlossen wird bzw. die Stege der in der Außenzelle angeordneten zungenförmigen Zelle aufeinander zu bewegt werden. Dadurch wird das Konkrement eingeklemmt.

Insgesamt wird durch die Rautenform der Außenzelle zusammen mit der Zelle-in-Zelle Anordnung die Verankerung des von innen heraus gehaltenen Konkrements am Funktionselement verbessert.

Alle Zellen der Gitterstruktur, also nicht nur die Außenzellen, sondern auch die Zellen des Schneidbereichs können rautenförmig sein. Die vorstehend beschriebene Kinematik der Gitterstruktur sowie das Crimpverhalten werden dadurch weiter verbessert. Die Gitterstruktur mit den rautenförmigen Zellen bzw. Maschen verfügt überdies über eine erhöhte Radialkraft und ist daher besonders gut zum tiefen Einschneiden oder Eindrücken des Konkrements bei der Expansion des im Inneren des Konkrements entlassenen Funktionselements geeignet.

Die zungenförmigen Zelle mit der Spitze ist im expandierten Zustand des Funktionselements und in der Ruhelage in der zylindrischen Mantelfläche der Gitterstruktur angeordnet. Im Gebrauch ist die zungenförmigen Zelle mit der Spitze radial nach außen und/oder radial nach innen aus der zylindrischen Mantelfläche auslenkbar derart, dass die zungenförmige Zelle mit der Spitze zum Halten des Konkrements über die Mantelfläche der Gitterstruktur vorsteht.

Dadurch, dass die zungenförmigen Zelle im Ruhezustand in der zylindrischen Mantelfläche der Gitterstruktur angeordnet ist, lässt sich das Funktionselement gut crimpen sowie einfach und sicher durch den Katheter in das Hohlgefäß einführen. Im Gefäß kann die zungenförmigen Zelle durch eine Längskrümmung des Gittergeflechts radial nach außen ausgelenkt werden, so dass die Spitze der zungenförmigen Zelle und damit auch ein Großteil der zungenförmigen Zelle selbst bzw. die komplette zungenförmigen Zelle zum Halten des Konkrements über die Mantelfläche vorsteht. Die Auslenkung der zungenförmigen Zelle wird dadurch weiter verbessert, dass die Gitterzelle oder Masche, in der die zungenförmige Zelle angeordnet ist, flexibler als die Gitterzellen und/oder Maschen des umgebenden Schneidbereichs ist. Wenn die zungenförmige Zelle bei der Längskrümmung auf dem Außenradius des Funktionselements angeordnet ist, wird erreicht, dass die zungenförmige Zelle mit dem Konkrement in Eingriff bleibt, wenn das Funktionselement einer Gefäßkrümmung folgt. Wenn mehrere zungenförmige Zellen auf dem Umfang des Funktionselements verteilt angeordnet sind, wird die Anordnung auf dem Außenradius bei einer Längskrümmung, also bei einer Krümmung um eine im Wesentlichen quer zur Längsachse des Funktionselements verlaufende Achse, unabhängig von der Krümmungsrichtung erreicht.

Außerdem kann zusätzlich oder alternativ die zungenförmige Zelle bzw. Zunge radial nach innen auslenkbar sein, insbesondere durch das Konkrement, das sich aufgrund des Schneidbereichs der Gitterstruktur, der die zungenförmige Zelle zumindest bereichsweise umgibt, ins Lumen des Funktionselements hineinbewegt. Dabei nimmt das Konkrement die zungenförmige Zelle mit, so dass diese radial nach innen bewegt wird. Die nach innen ausgelenkte zungenförmige Zelle bildet ein Widerlager für das Konkrement, durch das Zug- bzw. Druckkräfte (in Abhängigkeit von der Orientierung und Bewegungsrichtung der zungenförmige Zelle) übertragen werden können. Dadurch wird die Verankerung des Konkrements im Funktionselement verbessert.

Bei einer bevorzugten Ausführung ist vorgesehen, dass die Gitterzelle oder Masche, in der das Halteelement angeordnet ist, flexibler als die Gitterzellen und/oder Maschen des Schneidbereichs ist, der das Halteelement zumindest bereichsweise, insbesondere vollständig umgibt.

Durch die unterschiedliche Flexibilität der Gitterzelle oder Masche, in der die zungenförmige Zelle angeordnet ist, und der umgebenden Gitterstruktur des Schneidbereichs wird die Wirkung verbessert, dass einerseits die zungenförmige Zelle ausgelenkt und andererseits der Schneidbereich in das Gewebe des Konkrements eindringen kann, wodurch sich die gewünschte Änderung der Relativlage zwischen der zungenförmige Zelle und dem Schneidbereich einstellt.

Die Flexibilität des Halteelements relativ zu den umgebenden Zellen bzw. Maschen kann bei einer bevorzugten Ausführung dadurch beeinflusst werden, dass die Gitterzelle oder Masche, in der das Halteelement angeordnet ist, kleiner, gleich groß oder größer als die umgebenden Gitterzellen oder Maschen des Schneidbereichs sind.

Insbesondere dann, wenn die Gitterzelle bzw. Masche mit dem Halteelement größer ist, als die umgebenden Gitterzellen oder Maschen des Schneidbereichs, wird die axiale Verformbarkeit der größeren Zelle erhöht. Dies führt dazu, dass eine Zellenreihe, die aus größeren Zellen besteht, sich besser biegt und damit eine erhöhte Flexibilität aufweist. In Kombination mit dem Halteelement führt dies dazu, dass das Halteelement sich bei der Biegung, insbesondere bei der Längsbiegung des Funktionselements nach außen aufstellt. Die Größe der Zelle bezieht sich auf deren Breite bzw. Höhe. Alternativ kann sich die Größe auch auf die Zellfläche beziehen.

Alle Haltelemente können in distaler Richtung ausgerichtet sein derart, dass die Spitzen in distaler Richtung weisen.

Das Verhältnis der Steglänge der Gitterzelle oder Masche mit dem Halteelement zu der Steglänge der umgebenden Gitterzellen oder Maschen des Schneidbereichs kann mindestens 110%, insbesondere mindestens 120%, mindestens 130%, mindestens 140%, mindestens 150%, mindestens 175%, mindestens 200% betragen. Die Halteelemente können Stege aufweisen, deren Stegbreite höchstens 150%, insbesondere höchstens 120%, höchstens 110%, höchstens 90%, höchstens 80%, höchstens 70%, höchstens 60%, höchstens 50%, höchstens 40% der Stegbreite der umgebenden Gitterzellen oder Maschen des Schneidbereichs aufweisen. Bei einem weiteren bevorzugten Ausführungsbeispiel beträgt das Verhältnis der Stegbreite der Stege einer Gitterzelle oder Masche mit einem Halteelement zur Stegbreite der umgebenden Gitterzellen oder Maschen des Schneidbereichs höchstens 200%, insbesondere höchstens 175%, höchstens 50%, höchstens 20%, höchstens 110%, höchstens 90%, höchstens 80%, höchstens 70%, höchstens 60%, höchstens 50% der Stegbreite.

Bei einem weiteren bevorzugten Ausführungsbeispiel ist in Längsrichtung der Gitterstruktur wenigstens ein Ringsegment mit einem oder mit mehreren Halteelementen und wenigstens ein Ringsegment ohne Halteelement angeordnet. Das Verhältnis der Zellenanzahl eines Ringsegments mit Halteelement zur Zellenanzahl eines Ringelements ohne Halteelement kann höchstens 3/4, insbesondere 2/3, höchstens 1/2, höchstens 1/3 betragen. Das Verhältnis der Anzahl zwischen Ringelemente mit wenigstens einem Halteelement zur Anzahl von Ringelementen ohne Halteelement kann höchstens 1:3, insbesondere höchstens 1:2, höchstens 2:3, höchstens 1:1, höchstens 3:2, höchstens 2:1, höchstens 3:1, höchstens 4:1 betragen.

Die Ringsegmente mit wenigstens einem Halteelement und die Ringsegmente ohne Halteelement können sich in Längsrichtung des Gittergeflechts abwechseln. Ein bevorzugtes Muster ist die alternierende Anordnung eines Ringsegments mit einem oder mehreren Halteelementen und eines Ringsegments ohne Halteelemente. Andere alternierende Muster sind möglich, wie beispielsweise:
ein Ringsegment mit wenigstens einem Halteelement/zwei Ringsegmente ohne Halteelement oder umgekehrt,
ein Ringsegment mit wenigstens einem Halteelement/drei Ringsegmente ohne Halteelement und umgekehrt und
zwei Ringsegmente jeweils mit wenigstens einem Halteelement/drei Ringsegmente jeweils ohne Halteelement und umgekehrt.

Mit Blick auf das Verhältnis der Anzahl von Ringsegmenten mit wenigstens einem Halteelement und einem Ringsegment ohne Halteelement sind folgende konkrete Ausführungsformen denkbar. In Längsrichtung der Vorrichtung können mindestens 1, insbesondere mindestens 2, mindestens 3, mindestens 4, mindestens 5, mindestens 6 Ringelemente mit wenigstens einem Halteelement oder Ringelemente ohne Halteelement vorgesehen sein. In Längsrichtung der Vorrichtung können höchstens 6, insbesondere höchstens 5, insbesondere höchstens 4, insbesondere höchstens 3, insbesondere höchstens 2, insbesondere höchstens 1 Ringelemente mit wenigstens einem Halteelement oder Ringelemente ohne Halteelement vorgesehen sein. Die vorgenannten Ober- und Untergrenzen können zur Bildung von Bereichen jeweils miteinander kombiniert werden.

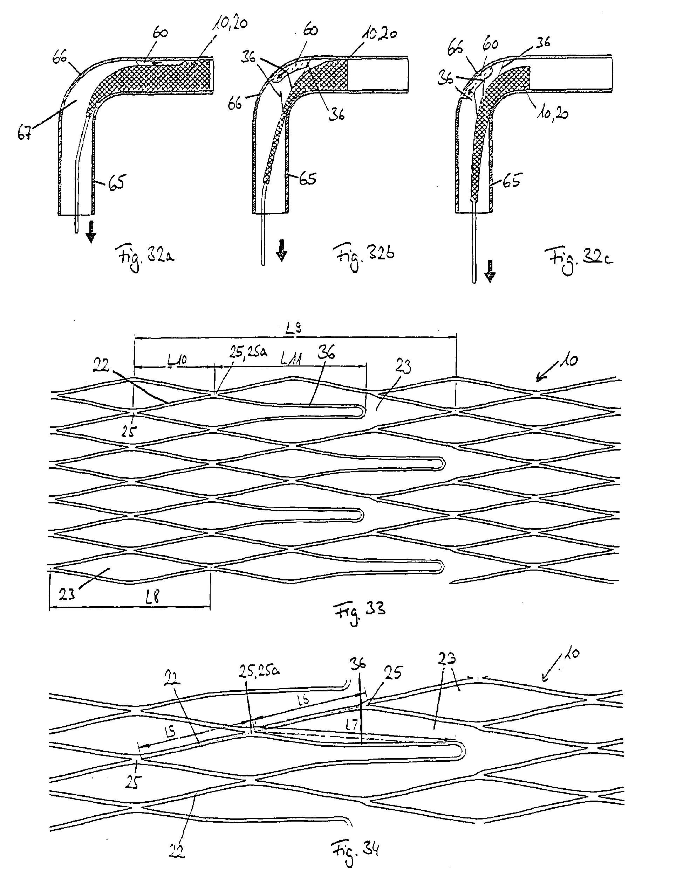

Innerhalb eines Ringsegments können Gitterzellen oder Maschen mit jeweils wenigstens einem Halteelement und Gitterzellen oder Maschen ohne Halteelement in axialer Richtung zueinander versetzt angeordnet sein (beispielsweise Fig. 33 bis 35), insbesondere wenn die Zellanzahl des Ringelements mit wenigstens einem Halteelement von der Zellenanzahl des Ringelements ohne Halteelement abweicht, beispielsweise aufgrund der unterschiedlichen Größe. Die Verbindungslinie der Schwerpunkte bzw. Mittelpunkte der Gitterzellen beschreibt in Umfangsrichtung eine Zickzackform.

Der Erfindung liegt ferner der Gedanke zu Grunde, eine medizinische Vorrichtung zum Entfernen von Konkrementen aus Körperhohlorganen mit einem Funktionselement anzugeben, das eine rotationssymmetrische Gitterstruktur und ein Mittel zum Halten eines Konkrements in der Gitterstruktur aufweist, und mit einem Katheter zum Zuführen und Entfernen des Funktionselements aus dem Körper, wobei das Funktionselement von einem komprimierten Zustand im Katheter in einen expandierten Zustand außerhalb des Katheters überführbar ist, in welchem das Funktionselement distal vom Katheter angeordnet ist. Die Gitterstruktur weist einen Schneidbereich mit Stegen auf, die im Gebrauch das Konkrement bei der Überführung des Funktionselements vom komprimierten Zustand in den expandierten Zustand zumindest teilweise radial durchdringen. Das Mittel zum Halten umfasst wenigstens ein Halteelement, das einerseits mit der Gitterstruktur und andererseits mit einem im Katheter angeordneten Führungsdraht verbunden ist derart, dass das Halteelement im expandierten Zustand des Funktionselements radial nach innen ausgelenkt ist.

Die Erfindung beruht auf einem grundlegend anderen Konzept als das bekannte Thrombektomiesytem, bei dem das Fangelement an der Gefäßwand anliegt und der Thrombus durch Aspiration in das proximal gelegenen Fangelement eingesaugt wird. Durch den Schneidbereich der Gitterstruktur ist es bei der erfindungsgemäßen Vorrichtung vielmehr möglich, die Gitterstruktur bzw. allgemein das Funktionselement innerhalb des Konkrements zu expandieren. Die Stege des Schneidbereichs durchdringen das Konkrement dabei in radialer Richtung. Die Stege des Schneidbereichs können sich im Konkrement verankern, so dass ein Kontakt mit der Gefäßwand des Körperhohlorgans vermieden wird. Auf diese Weise wird die Belastung für die Gefäßwand beim Einsatz der Vorrichtung reduziert. Ferner weist die erfindungsgemäße Vorrichtung eine verbesserte Handhabbarkeit und Anwendungssicherheit auf. Insbesondere die Anwendungssicherheit wird durch die mit dem Führungsdraht verbundenen Halteelemente erhöht. Während die Stege des Schneidbereichs in das Konkrement radial vordringen, werden die Halteelemente radial nach innen ausgelenkt und bilden somit eine Verbindung zwischen dem Führungsdraht und der Wandungsebene der Gitterstruktur. Durch die Verbindung mit dem Führungsdraht werden die Halteelemente beim Expandieren zwangsgeführt. Die radial nach innen ausgelenkten Halteelemente gewährleisten eine Verankerung des Konkrements im Funktionselement, selbst wenn die Stege des Schneidbereichs das Konkrement vollständig durchdringen. Das Konkrement wird auf diese Weise sicher innerhalb des Funktionselements fixiert und kann einfach aus dem Körperhohlorgan entfernt werden. Der wirksame Querschnitt des Funktionselements wird durch die Halteelemente vergrößert.

Die Erfindung funktioniert im Prinzip mit einem einzigen Halteelement. Durch Anordnung mehrerer Halteelemente wird die Wirksamkeit der Erfindung erhöht.

Das Halteelement kann mit dem Führungsdraht fest oder axial längsverschieblich verbunden sein. Im Falle einer festen Verbindung zwischen dem Halteelement und dem Führungsdraht ist es vorteilhaft, wenn das Halteelement eine erhöhte Flexibilität bzw. Elastizität aufweist, um die Längen- und Durchmesseränderung des Funktionselements bei der Expansion auszugleichen. Der Vorteil einer festen Verbindung zwischen dem Halteelement und dem Führungsdraht besteht darin, dass der Expansionsgrad des Funktionselements bereichsweise, insbesondere punktuell an der Verbindungsstelle des Halteelements mit der Gitterstruktur, einstellbar ist. Alternativ kann die Längen- und Durchmesseränderung des Funktionselements bei der Expansion durch eine axial verschiebliche Lagerung des Halteelements am Führungsdraht erreicht werden. Durch die axial verschiebliche Lagerung wird ferner erreicht, dass sich das Funktionselement an die Struktur des Konkrements individuell anpassen kann.

Bei einer bevorzugten Ausführungsform der erfindungsgemäßen medizinischen Vorrichtung weist das Halteelement eine Spitze auf, die mit dem Führungsdraht verbunden ist. Konkret kann sich das Halteelement im expandierten Zustand des Funktionselements von der Umfangsebene der Gitterstruktur bis zum Führungsdraht erstrecken.

Die Spitze kann eine Öse oder eine Schlaufe oder eine Gleithülse aufweisen, durch die der Führungsdraht geführt ist. Eine derartige Konstruktion der Spitze ist besonders einfach herstellbar. Durch die Öse, die Schlaufe oder die Gleithülse ist eine einfache und zuverlässige axialverschiebliche Verbindung zwischen dem Halteelement und dem Führungsdraht ermöglicht.

Die Spitze kann ferner eine vergrößerte, insbesondere löffelartig ausgebildete, Kontaktfläche aufweisen. Die Kontaktfläche verbessert die Verankerung des Halteelements innerhalb eines Konkrements. Durch die Auslenkung des Halteelements im expandierten Zustand des Funktionselements wird vorteilhafterweise eine im Wesentlichen radiale Ausrichtung der Kontaktfläche der Spitze des Halteelements bewirkt. Die Kontaktfläche wirkt somit beim Bewegen des Konkrements mit dem Konkrement zusammen und erhöht damit die Fixierung des Konkrements innerhalb des Funktionselements.

Die folgenden Ausführungsformen werden auch im Zusammenhang mit der Vorrichtung gemäß Anspruch 1 offenbart, bei der die Verbindung des Halteelements bzw. der Halteelemente mit dem Führungsdraht nicht zwingend ist.

Vorzugsweise ist das Halteelement im Wesentlichen V-förmig ausgebildet. Das Halteelement umfasst zwei Speichen, die mit jeweils wenigstens einem Steg des Schneidbereichs verbunden sind. Eine derartige V-förmige Struktur mit Speichen ist im Rahmen der Herstellung von medizinischen Gitterstrukturen einfach möglich. Das Halteelement kann auf diese Weise einfach hergestellt bzw. in die medizinische Gitterstruktur integriert werden.

Die Speichen des Halteelements können einen kleinere mechanische Steifigkeit, insbesondere eine kleinere Breite und/oder Dicke, als die Stege des Schneidbereichs aufweisen. Im Allgemeinen ist vorgesehen, dass das Halteelement im Vergleich zu den Stegen des Schneidbereichs eine erhöhte Flexibilität bzw. reduzierte mechanische Steifigkeit aufweist. Die erhöhte Flexibilität kann beispielsweise durch die Breite bzw. Dicke der Speichen eingestellt werden. Alternativ oder zusätzlich kann die erhöhte Flexibilität des Halteelements bzw. der Speichen durch Materialeigenschaften beeinflusst werden. Weitere Faktoren, die die Flexibilität des Halteelements bzw. der Speichen des Halteelements im Vergleich zu den Stegen des Schneidbereichs beeinflussen können, sind beispielsweise der Anlenkwinkel zwischen den Speichen des Halteelements und den Stegen des Schneidbereichs und/oder die Länge der Speichen im Vergleich zu den Stegen des Schneidbereichs. In allen möglichen Konfigurationen bewirkt die erhöhte Flexibilität bzw. verringerte mechanische Steifigkeit ein verbessertes Auslenkverhalten des Halteelements. Konkret ist das Halteelement derart angepasst, dass beim Durchdringen des Konkrements durch das Funktionselement das Halteelement radial nach innen ausgelenkt wird. Die Auslenkung des Halteelements kann also nicht nur durch die Verbindung mit dem Führungsdraht, sondern auch durch den Druck des Konkrementmaterials erfolgen. Die Stege des Schneidbereichs sind hingegen derart steif ausgebildet, dass die Stege in das Material des Konkrements eindringen.

Bei einer bevorzugten Ausführungsform ist vorgesehen, dass die Gitterstruktur wenigstens ein Fängerelement umfasst, das im expandierten Zustand radial nach außen über die Gitterstruktur vorsteht. Das Fängerelement hat den Vorteil, dass die sich zwischen der Gitterstruktur und der Gefäßwand befindenden Teile des Konkrements vom Fängerelement mitgenommen werden, wenn das Funktionselement in den Katheter zurückgezogen wird.

Das Halteelement kann sich im komprimierten Zustand des Funktionselements in längsaxialer Richtung des Funktionselements erstrecken. Auf diese Weise ist das Halteelement gut in die Gitterstruktur integriert, so dass sich ein relativ kleiner, komprimierter Querschnittsdurchmesser des Funktionselements einstellen lässt. Es ist jedoch nicht ausgeschlossen, dass sich das Halteelement im komprimierten Zustand des Funktionselements in Umfangsrichtung erstreckt.

Vorteilhafterweise sind mehrere Halteelemente vorgesehen. Die Verankerungsfunktion des Funktionselements in Bezug auf das zu entfernende Konkrement wird somit weiter verbessert. Die mehreren Halteelemente können unterschiedliche Längen aufweisen. Auf diese Weise können Halteelemente, die im Wesentlichen im gleichen axialen Abschnitt der rotationssymmetrischen Gitterstruktur mit den Stegen des Schneidbereichs verbunden sind, an unterschiedlichen Stellen mit dem Führungsdraht verbunden sein, wodurch sich die Handhabbarkeit und Anwendungssicherheit der medizinischen Vorrichtung erhöht. Ferner kann durch die unterschiedlichen Längen der mehreren Halteelemente gewährleistet werden, dass der Führungsdraht im expandierten Zustand des Funktionselements zentriert innerhalb des Funktionselements verläuft.

Des Weiteren können mehrere Halteelemente vorgesehen sein, die an einem axialen Ende des Funktionselements in Umfangsrichtung benachbart angeordnet sind. Die mehreren Halteelemente können mit dem Führungsdraht verbunden sein, um einen netzartigen Verschluss des Funktionselements zu bilden. Konkret können die mehreren Halteelemente in dem selben axialen Endabschnitt der Gitterstruktur bzw. des Funktionselements angeordnet sein. Die Spitzen der Halteelemente können mit dem Führungsdraht verbunden sein, so dass das axiale Ende der Gitterstruktur bei der Expansion des Funktionselements geschlossen bleibt. Auf diese Weise kann bei der Expansion des Funktionselements eine vollständig geschlossene Gitterstruktur gebildet werden, wodurch die Einkapselung eines Konkrements verbessert wird.

Bei einer weiteren bevorzugten Ausführungsform der medizinischen Vorrichtung umfasst der Führungsdraht wenigstens ein Begrenzungselement. Das Begrenzungselement kann im expandierten Zustand des Funktionselements eine axiale Bewegung des Halteelements, insbesondere der Spitze, begrenzen. Durch die Begrenzung der Axialbewegung der Spitze des Halteelements wird gleichzeitig eine Begrenzung der möglichen Expansion des Funktionselements erreicht. Durch entsprechende Anordnung des Begrenzungselements kann also die maximal mögliche Expansion des Funktionselements eingestellt werden. Beispielsweise kann die maximal mögliche Expansion des Funktionselements derart eingestellt werden, dass das Funktionselement die Gefäßwand des zu behandelnden Körperhohlorgans nicht berührt. Verletzungen der Gefäßwand werden somit effektiv vermieden.

Das Begrenzungselement kann eine Nut, die in dem Führungsdraht ausgebildet ist, oder eine Hülse umfassen, die fest mit dem Führungsdraht verbunden ist. Eine derartige Gestaltung des Begrenzungselements ist besonders einfach herstellbar.

Der Führungsdraht kann ein flexibles Element umfassen. Das flexible Element kann derart angepasst sein, dass der Führungsdraht längsaxial verlängerbar ist. Das flexible Element kann die Längenänderung, insbesondere die Verkürzung des Funktionselements bzw. der Gitterstruktur bei der Expansion, kompensieren. Eine derartige Längenänderung bzw. Verkürzung der Gitterstruktur wird im Allgemeinen als "Foreshortening" bezeichnet. Das flexible Element kann ferner eine Vorspannkraft aufweisen, so dass bei der Entlassung des Funktionselements aus dem Katheter die Expansion durch die Vorspannkraft des flexiblen Elements des Führungsdrahts unterstützt wird. Der Führungsdraht ist vorzugsweise mit dem Funktionselement fest verbunden. Insbesondere kann der Führungsdraht mit einem distalen Ende und/oder einem proximalen Ende des Funktionselements fest verbunden sein.

Gemäß einer weiteren bevorzugten Ausführungsform der erfindungsgemäßen medizinischen Vorrichtung weist das Funktionselement wenigstens ein Aufstellelement auf, das bei einer geradlinigen Anordnung des Funktionselements bündig in der Gitterstruktur angeordnet ist. Bei einer längsgekrümmten Anordnung des Funktionselements ist das Aufstellelement radial nach außen ausgelenkt. Konkret ist vorgesehen, dass das Aufstellelement bündig in der Wandungsebene der Gitterstruktur angeordnet ist unabhängig davon, ob die Gitterstruktur den komprimierten oder expandierten Zustand einnimmt. Die bündige Anordnung des Aufstellelements erfolgt generell in geradliniger Anordnung des Funktionselements. Das bedeutet, dass sich eine Längsachse des Funktionselements bzw. der rotationssymmetrischen Gitterstruktur geradlinig erstreckt. Das Aufstellelement ist also bündig in der Gitterstruktur angeordnet, wenn das Funktionselement bzw. die Gitterstruktur im Wesentlichen eine Zylinderform aufweist. Bei einer Krümmung des Funktionselements in Längsrichtung erfolgt hingegen ein Aufstellen des Aufstellelements in eine radial nach außen weisende Richtung. Bei der Längskrümmung verlässt das Funktionselement bzw. die Gitterstruktur die Zylinderform und geht in eine gebogene Form, ähnlich einer Rohrkrümmung, über.

Das Aufstellelement kann auf der Seite der Gitterstruktur angeordnet sein, die den größeren Krümmungsradius aufweist. Vorzugsweise ist das Aufstellelement derart angepasst, dass eine Auslenkung nur dann erfolgt, wenn das Aufstellelement auf der Seite der Gitterstruktur angeordnet ist, die die den größeren Krümmungsradius bezogen auf die Längsachse der Gitterstruktur aufweist.

Durch die Krümmung des Funktionselements bzw. der Gitterstruktur wird das Aufstellelement radial nach außen ausgelenkt. Bei der Anordnung des Funktionselements in Gefäßkrümmungen besteht die Gefahr, dass zwischen einer Gefäßwand und der Gitterstruktur ein Freiraum gebildet wird, so dass sich Konkrementpartikel von dem Funktionselement lösen können. Dies wird durch das Aufstellelement verhindert, das in einer Gefäßkrümmung radial nach außen ausgelenkt ist und somit in den gebildeten Freiraum hineinragt. Die zuverlässige Entfernung eines Konkrements aus einem Körperhohlorgan wird somit verbessert.

Die Erfindung wird im Folgenden anhand von Ausführungsbeispielen unter Bezugnahme auf die beigefügten, schematischen Zeichnungen näher erläutert. Darin zeigen:
- Fig. 1: eine Draufsicht auf einen Ausschnitt der Gitterstruktur eines Funktionselements der erfindungsgemäßen medizinischen Vorrichtung nach einem bevorzugten Ausführungsbeispiel;
- Fig. 2a: eine Seitenansicht eines Funktionselements der erfindungsgemäßen medizinischen Vorrichtung gemäß einem bevorzugten Ausführungsbeispiel;
- Fig. 2b: einen Querschnitt des Funktionselements gemäß Fig. 2a;
- Fig. 3a: eine Seitenansicht eines Funktionselements der erfindungsgemäßen medizinischen Vorrichtung gemäß einem weiteren bevorzugten Ausführungsbeispiel;
- Fig. 3b: einen Querschnitt des Funktionselements gemäß Fig. 3a;
- Fig. 4a-4b: jeweils eine Seitenansicht der erfindungsgemäßen medizinischen Vorrichtung nach einem bevorzugten Ausführungsbeispiel in unterschiedlichen Phasen der Entlassung des Funktionselements in einem Blutgefäß;
- Fig. 5: eine Draufsicht auf ein Detail einer Gitterstruktur des Funktionselements der erfindungsgemäßen medizinischen Vorrichtung nach einem bevorzugten Ausführungsbeispiel;
- Fig. 6-9: jeweils eine Detailansicht einer Zellendecke der Struktur des Funktionselements der erfindungsgemäßen medizinischen Vorrichtung nach einem bevorzugten Ausführungsbeispiel;
- Fig. 10: eine Draufsicht auf eine Gitterstruktur des Funktionselements der erfindungsgemäße medizinischen Vorrichtung nach einem weiteren bevorzugten Ausführungsbeispiel;
- Fig. 11a-11d: jeweils eine Abwicklung eines Funktionselements der erfindungsgemäßen medizinischen Vorrichtung nach einem der bevorzugten Ausführungsbeispiel;
- Fig. 12a: eine Seitenansicht eines Funktionselements der erfindungsgemäßen medizinischen Vorrichtung gemäß einem weiteren bevorzugten Ausführungsbeispiel;
- Fig. 12b: einen Querschnitt des Funktionselements gemäß Fig. 12a;
- Fig. 13: eine Seitenansicht eines Funktionselements der erfindungsgemäßen medizinischen Vorrichtung gemäß einem weiteren bevorzugten Ausführungsbeispiel;

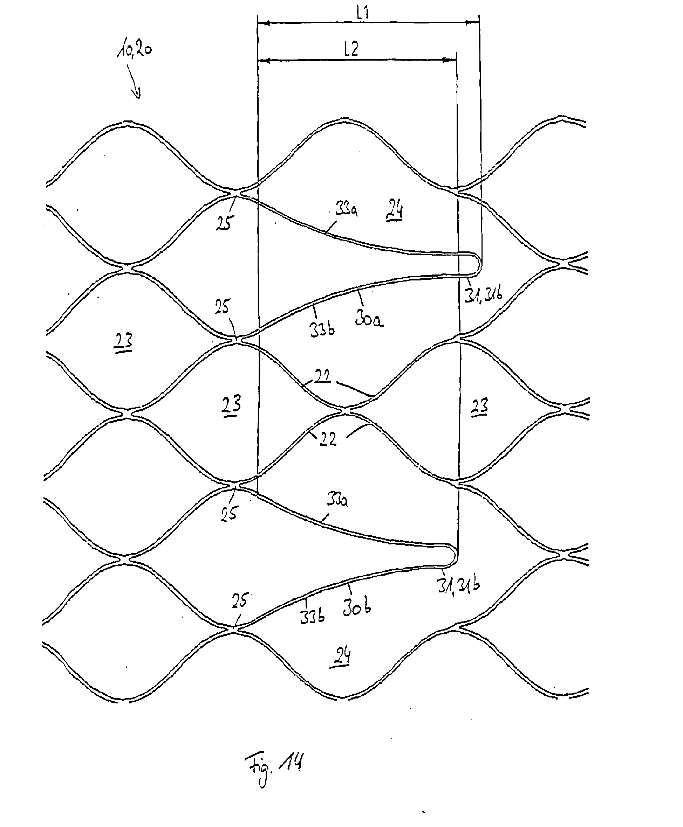

- Fig. 14: eine Draufsicht auf eine Gitterstruktur des Funktionselements der erfindungsgemäßen medizinischen Vorrichtung gemäß einem weiteren bevorzugten Ausführungsbeispiel;

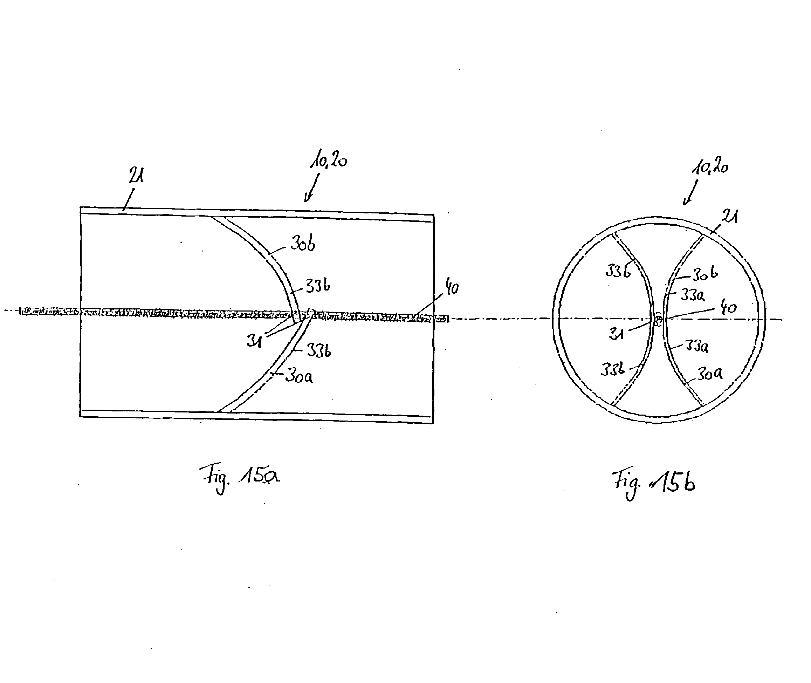

- Fig. 15a: eine Seitenansicht eines Funktionselements der erfindungsgemäßen medizinischen Vorrichtung nach einem weiteren bevorzugten Ausführungsbeispiel;
- Fig. 15b: einen Querschnitt des Funktionselements gemäß Fig. 15a;
- Fig. 16a-16c: jeweils eine Abwicklung eines Funktionselements der erfindungsgemäßen medizinischen Vorrichtung nach einem bevorzugten Ausführungsbeispiel;
- Fig. 17a: eine Abwicklung einer Gitterstruktur des Funktionselements der erfindungsgemäßen medizinischen Vorrichtung nach einem bevorzugten Ausführungsbeispiel;
- Fig. 17b: einen Querschnitt des Funktionselements gemäß Fig. 17a;
- Fig. 18a: eine Abwicklung einer Gitterstruktur des Funktionselements einer erfindungsgemäßen medizinischen Vorrichtung gemäß einem weiteren bevorzugten Ausführungsbeispiel;
- Fig. 18b: einen Querschnitt des Funktionselements gemäß Fig. 18a;
- Fig. 19a: eine Abwicklung einer Gitterstruktur des Funktionselements der erfindungsgemäßen medizinischen Vorrichtung gemäß einem weiteren bevorzugten Ausführungsbeispiel;
- Fig. 19b: eine Querschnittsansicht des Funktionselements gemäß Fig. 19a;
- Fig. 20a-20d: jeweils eine Seitenansicht eines Funktionselements der erfindungsgemäßen medizinischen Vorrichtung gemäß einem bevorzugten Ausführungsbeispiel im Gebrauch;
- Fig. 21a: eine Seitenansicht eines Funktionselements der erfindungsgemäßen medizinischen Vorrichtung gemäß einem bevorzugten Ausführungsbeispiel in einem teilexpandierten Zustand;
- Fig. 21b: eine Seitenansicht des Funktionselements der erfindungsgemäßen medizinischen Vorrichtung gemäß Fig. 21a in einem vollexpandierten Zustand;
- Fig. 22a: eine Seitenansicht eines Funktionselements der erfindungsgemäßen medizinischen Vorrichtung gemäß einem weiteren bevorzugten Ausführungsbeispiel im teilexpandierten Zustand;
- Fig. 22b: eine Seitenansicht des Funktionselements der erfindungsgemäßen medizinischen Vorrichtung gemäß Fig. 22a in einem vollexpandierten Zustand;
- Fig. 23a: eine Seitenansicht eines Funktionselements der erfindungsgemäßen medizinischen Vorrichtung gemäß einem weiteren bevorzugten Ausführungsbeispiel in einem teilexpandierten Zustand;
- Fig. 23b: eine Seitenansicht des Funktionselements der erfindungsgemäßen medizinischen Vorrichtung gemäß Fig. 23a in einem vollexpandierten Zustand;
- Fig. 24a: eine Seitenansicht der erfindungsgemäßen medizinischen Vorrichtung gemäß einem weiteren bevorzugten Ausführungsbeispiel in einem teilexpandierten Zustand des Funktionselements; und
- Fig. 24b: eine Seitenansicht der Vorrichtung gemäß Fig. 29a in einem vollexpandierten Zustand des Funktionselements.
- Fig. 25a: eine schematische Seitenansicht der Vorrichtung nach einem erfindungsgemäßen Ausführungsbeispiel mit radial nach außen ausgelenkten Fängerelementen;
- Fig. 25b: einen Querschnitt durch die Vorrichtung gemäß Fig. 25a;
- Fig. 25c: eine Abwicklung der Gitterstruktur der Vorrichtung gemäß Fig. 25a;
- Fig. 26a-26c: eine Vorrichtung nach einem weiteren erfindungsgemäßen Ausführungsbeispiel mit Fängerelementen im Gebrauch in verschiedenen Stadien der Entfernung eines Konkrements aus einem Gefäß;
- Fig. 26d: einen Querschnitt durch die Vorrichtung gemäß Fig. 26c und des Gefäßes, in dem die Vorrichtung angeordnet ist;
- Fig. 27a, 27b: eine Draufsicht auf verschiedene Varianten des Fängerelements;
- Fig. 28a, 28b: Seitenansichten verschiedener Varianten des Fängerelements mit gebogenem freien Ende;
- Fig. 29: eine Draufsicht auf ein Fängerelement nach einem weiteren Ausführungsbeispiel mit unterschiedlichen Funktionsabschnitten;
- Fig. 30a-30d: Seitenansichten einer Vorrichtung nach einem erfindungsgemäßen Ausführungsbeispiel mit einem Fängerelement in verschiedenen Herstellungsstadien;
- Fig. 31: eine Draufsicht bzw. Seitenansicht eines Dorns zur Formgebung des Fängerelements;
- Fig. 32a-32c: Seitenansichten einer Vorrichtung nach einem erfindungsgemäßen Ausführungsbeispiel mit Aufstellelementen in unterschiedlichen Stadien bei der Entfernung des Konkrements;
- Fig. 33: eine Abwicklung der Gitterstruktur des Funktionselements gemäß Fig. 32a-32c;
- Fig. 34: eine Detailansicht der Abwicklung gemäß Fig. 33; und
- Fig. 35: eine Abwicklung eines Funktionselements der erfindungsgemäßen Vorrichtung nach einem bevorzugten Ausführungsbeispiel.

Die folgenden Ausführungen beziehen sich sowohl auf die Vorrichtung, bei der das Halteelement 30 mit dem Führungsdraht verbunden ist, als auch auf die Vorrichtung, bei der das Halteelement 30 nicht direkt mit dem Führungsdraht, sondern nur mit dem Gittergeflecht verbunden ist. Insbesondere die Ausbildung des Gittergeflechts sowie die Form und Funktionsweise des Halteelements, wie nachstehend beschrieben, bezieht sich auch auf die Vorrichtung gemäß Anspruch 1.

Die in den folgenden Ausführungsbeispielen beschriebene Vorrichtung weist im Allgemeinen ein Funktionselement 10 auf, das in ein Körperhohlorgan, insbesondere ein Blutgefäß 65, einführbar und in dem Körperhohlorgan bzw. Blutgefäß 65 expandierbar ist. Dazu weist die medizinische Vorrichtung einen Katheter 50 auf, in dem das Funktionselement 10 im komprimierten Zustand längsverschieblich anordenbar ist. Im expandierten Zustand, also während der Behandlung eines Körperhohlorgans bzw. Blutgefäßes 65, ist das Funktionselement außerhalb des Katheters 50, insbesondere distal außerhalb des Katheters 50, angeordnet.

In diesem Zusammenhang wird darauf hingewiesen, dass die medizinischen Richtungsangaben "distal" und "proximal" den Anwender der medizinischen Vorrichtung als Bezugspunkt zu Grunde legen. Distal angeordnete Elemente oder Bereiche sind daher vom Anwender der medizinischen Vorrichtung weiter entfernt als proximal angeordnete Elemente bzw. Bereiche.

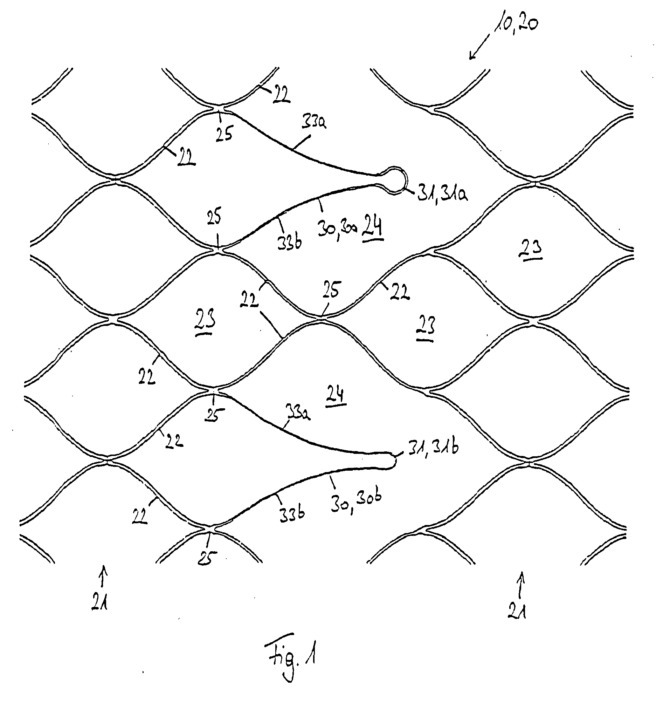

Um eine radiale Querschnittsänderung beim Übergang vom komprimierten Zustand in den expandierten Zustand zu ermöglichen, weist das Funktionselement 10 eine Gitterstruktur 20 auf. Ein Ausschnitt der Gitterstruktur 20 ist beispielhaft in Fig. 1 wiedergegeben. Die Gitterstruktur 20 umfasst Stege 22, die einen Schneidbereich 21 bilden. Die Stege 22 sind stellenweise miteinander verbunden. An den Verbindungsstellen der Stege 22 sind Knotenpunkte 25 gebildet.

Die Stege 22 begrenzen einerseits Zellen 23 und andererseits Maschen 24. Im Rahmen der Anmeldung werden Gitteröffnungen, die zu allen Seiten durch jeweils einen Steg 22 begrenzt sind, als Zellen bezeichnet. Mit anderen Worten weist eine Zelle 23 vier Seiten auf, die durch jeweils einen einzigen Steg 22 gebildet sind. Als Maschen 24 werden hingegen Gitteröffnungen bezeichnet, die wenigstens eine begrenzende Seite umfassen, die wenigstens zwei Stege 22 aufweist. Dabei verlaufen die wenigstens zwei Stege 22, die eine Seite der Masche 24 bilden, im Wesentlichen in gleicher Richtung.

Daraus ergibt sich, dass die durch Maschen 24 gebildeten Gitteröffnungen größer sind als die durch Zellen 23 gebildeten Gitteröffnungen. Sowohl die durch Maschen 24 als auch die durch Zellen 23 gebildeten Gitteröffnungen weisen eine allgemeine rautenförmige Kontur auf. Der Begriff "rautenförmig" ist nicht streng geometrisch zu verstehen, sondern umfasst auch die beispielsweise in Fig. 1 dargestellten Geometrien, bei denen die Seiten der Gitteröffnungen durch S-förmig gekrümmte Stege gebildet sind. Andere Formen der Gitteröffnungen sind möglich. Generell gilt, dass die Stege 22 der Zellen 23 bzw. der Maschen 24 in Bezug auf die Längsrichtung des Funktionselements 10 schräg angeordnet sind. Im Zusammenhang mit der Schneidfunktion der Stege hat die schräge Anordnung den Vorteil, dass die im Gebrauch in das Konkrement eingedrungenen Stege einen besseren Widerstand bieten, wenn das Konkrement zusammen mit dem Funktionselement 10 aus dem Gefäß entfernt wird, als dies der Fall wäre, wenn die Stege 22 beispielsweise parallel zur Längsrichtung des Funktionselements ausgerichtet wären.

Der Zellenwinkel ist groß, damit zum einen die Struktur flexibel ist und zum anderen die Zelle bei Überführung vom komprimierten in den expandierten Zustand stärker verformt wird. Durch die Verformung kann das Konkrement von den Zellen besser eingefangen werden. Das Konkrement wird sozusagen von den Zellen eingekniffen. Der Tip-Winkel in vollständig expandiertem Zustand ist größer als 70°, insbesondere größer als 80°, insbesondere größer als 90°, insbesondere größer als 110°, insbesondere größer als 120°, insbesondere größer als 130° .Um den Widerstand der Gitterstruktur 20 im Konkrement zu erhöhen, ist es ferner vorteilhaft, relativ viele Zellen 23 bzw. Maschen 24 vorzusehen, die in das Konkrement einschneiden. Beispielsweise können pro Ringsegment des Funktionselements 10 bzw. der Gitterstruktur 20 mehr als 4, insbesondere mehr als 6, mehr als 8, mehr als 12 Zellen vorgesehen sein. In Längsrichtung des Funktionselements 10 umfasst die Gitterstruktur 20 mehr als 2 Ringsegmente, insbesondere mehr als 3 Ringsegmente, insbesondere mehr als 4 Ringsegmente, insbesondere mehr als 5 Ringsegmente. Die Ringsegmente, die den Schneidbereich bilden, befinden sich in derselben zylindrischen Mantelfläche der Gitterstruktur 20.

Die Stege 22 bzw. die Zellen 23 der Gitterstruktur 20 bilden den Schneidbereich 21 der Gitterstruktur 20. Die Stegbreite im Bereich der Schneideelemente bzw. der Stege 22 und der Zellen 23 und/oder die Stegbreite der Halteelemente ist klein. Bei den Schneideelementen wird dadurch die Schneidefunktion begünstigt. Die Stegbreite der Schneideelemente und/oder der Halteelemente kann kleiner als 50 µm, insbesondere kleiner als 40 µm, insbesondere kleiner als 35 µm, insbesondere kleiner als 30 µm, insbesondere kleiner als 25 µm, insbesondere kleiner als 20 µm, insbesondere kleiner als 15µm sein.

Die vorstehende Beschreibung des Schneidbereichs 21 wird im Zusammenhang mit allen Ausführungsbeispielen dieser Anmeldung offenbart.

Bei dem Ausführungsbeispiel gemäß Fig. 1 weisen die Maschen 24 jeweils vier Seiten auf, die durch jeweils zwei Stege 22 gebildet sind. Dabei weisen die Stege 22 eine S-förmige Kontur auf, wobei sie an einer Seite der Masche 24 im Wesentlichen in gleicher Richtung ausgerichtet sind. Hingegen weisen die Zellen 23 gemäß Fig. 1 jeweils vier Seiten auf, die durch jeweils einen einzigen Steg 22 gebildet sind. Sowohl bei den Zellen 23, als auch bei den Maschen 24 weisen zwei Stege 22, die miteinander verbunden sind und unterschiedlichen Seiten der Zelle 23 bzw. Masche 24 angehören, eine unterschiedliche Orientierung auf.

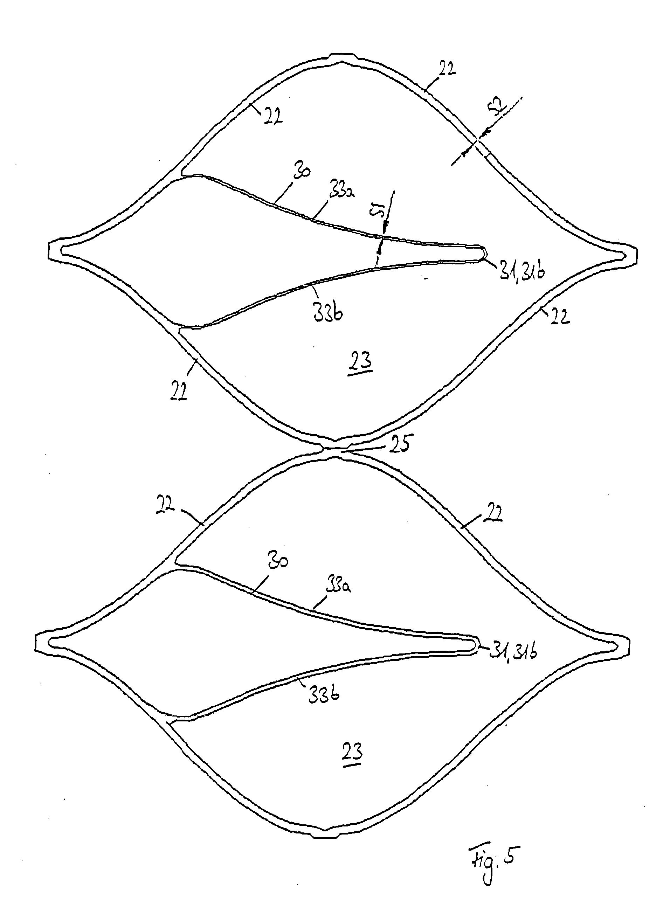

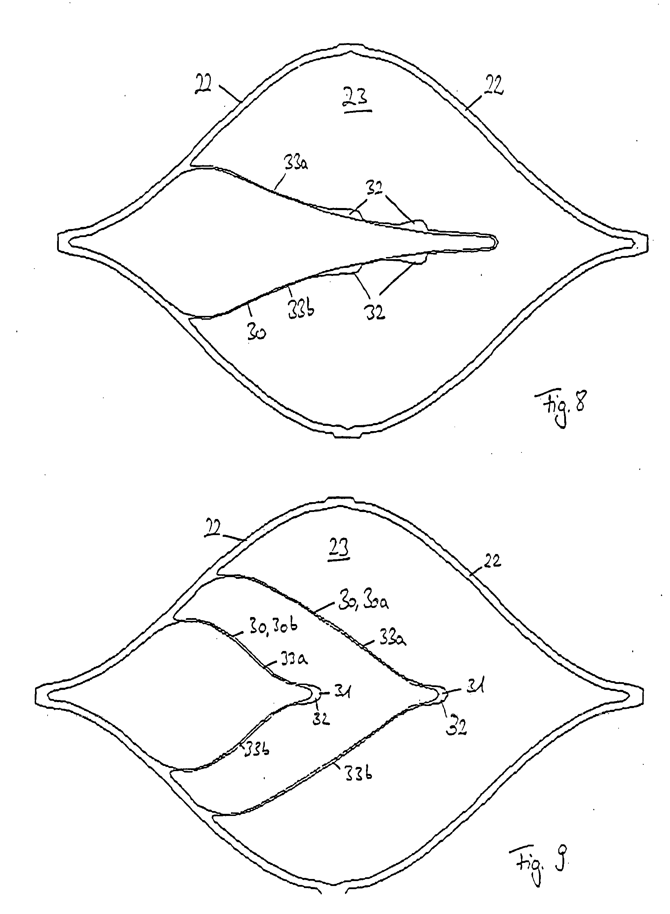

Bei dem Ausführungsbeispiel gemäß Fig. 1 ist in den Maschen 24 ferner jeweils ein Halteelement 30 angeordnet. Das Haltelement 30 bildet eine zungenförmige Zelle mit einer freien Spitze, die in die Masche 24 hinein ragt (Zelle-in-Zelle bzw. Zelle-in-Masche Anordnung). Es ist auch möglich, das Halteelement 30 in einer Zelle 23 anzuordnen (siehe Fig. 5, Fig. 9). Insbesondere ist in einer Masche 24 ein erstes Halteelement 30a und in einer weiteren Masche 24 ein zweites Halteelement 30b angeordnet. Die Halteelemente 30, 30a , 30b sind im Allgemeinen mit der Gitterstruktur 20, konkret mit den Stegen 22 der Gitterstruktur 20 verbunden. Insbesondere können die Halteelemente 30, 30a, 30b mit der Gitterstruktur 20 einteilig ausgebildet sein. Bei dem Ausführungsbeispiel gemäß Fig. 1 sind die Halteelemente 30, 30a, 30b mit jeweils zwei Knotenpunkten 25 der Gitterstruktur 20 verbunden. Insbesondere weisen die Halteelemente 30, 30a, 30b jeweils zwei Speichen 33a, 33b auf, die mit jeweils einem Knotenpunkt 25 verbunden sind. Die Speichen 33a, 33b bilden eine V-förmigen Verlauf des Halteelements 30. Das Halteelement 30 weist ferner eine Spitze 31 auf, in der die Speichen 33a, 33b miteinander verbunden sind.

Die Spitze 31 bildet ein Verbindungselement zwischen der Gitterstruktur 20 und einem Führungsdraht 40 (nicht dargestellt). Dazu kann die Gitterstruktur 20 unterschiedliche geometrische Ausgestaltungen aufweisen. Bei dem Ausführungsbeispiel gemäß Fig. 1 bildet die Spitze 31 des ersten Halteelements 30a eine Öse 31a, in der der Führungsdraht 40 axialverschieblich anordenbar ist. Die Öse 31a weist im Wesentlichen eine Kontur auf, die etwa der Kontur eines Dreiviertelkreises entspricht. Die Spitze 31 des zweiten Halteelements 30b bildet hingegen eine Schlaufe, deren Kontur im Wesentlichen der Kontur eines Halbkreises entspricht. In beiden Fällen ist die Spitze 31 des Halteelements 30 einteilig mit den Speichen 33a, 33b des Halteelements 30 ausgebildet. Die Spitze 31 kann auch ohne Verbindung zum Führungsdraht vorgesehen und somit frei beweglich sein.

Gegenüber dem Schneidbereich 21 bzw. den Stegen 22 der Gitterstruktur 20 weist das Halteelement 30 eine höhere Flexibilität bzw. Elastizität auf. Bei der Expansion des Funktionselements 10 wird das Halteelement 30 daher vergleichsweise einfach radial ausgelenkt. Das Halteelement 30 erstreckt sich im expandierten Zustand des Funktionselements 10 radial nach innen. Die Verbindung des Halteelements 30 mit dem Führungsdraht 40 gewährleistet die radiale Auslenkung des Haltelements 30 bei der Expansion des Funktionselements 10.

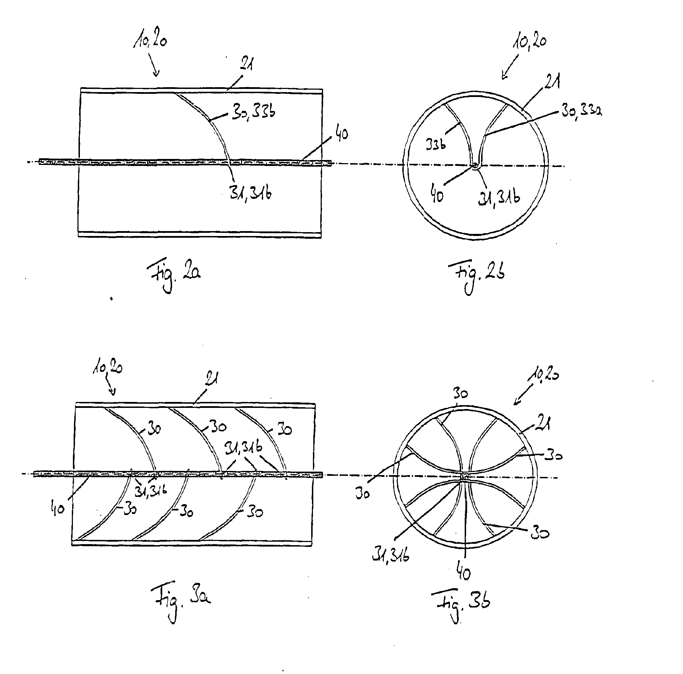

Die radiale Auslenkung des Halteelements 30 im expandierten Zustand des Funktionselements 10 ist in den Fig. 2a und 2b dargestellt. Dabei weist das Funktionselement 10 ein einziges Halteelement 30 auf, das im Wesentlichen V-förmig ausgebildet ist und eine als Schlaufe 31b ausgebildete Spitze 31 aufweist. In der Schlaufe 31b verläuft der Führungsdraht 40, der sich im Wesentlichen längsaxial durch das Funktionselement 10 hindurch erstreckt. Das Halteelement 30 kann mit dem Führungsdraht 40 axialverschieblich verbunden sein. Mit anderen Worten ist der Führungsdraht 40 in die Schlaufe 31b im Wesentlichen lose eingelegt. Die Spitze 31 des Halteelements 30 kann also auf dem Führungsdraht 40 gleiten. Alternativ kann die Spitze 31 mit dem Führungsdraht 40 fest verbunden sein. Eine axialverschiebliche Lagerung der Spitze 31 auf dem Führungsdraht 40 ist bevorzugt, wobei die Materialien bzw. Oberflächen des Führungsdrahts 40 und des Halteelements 30, insbesondere der Spitze 31, derart angepasst sein können, dass eine minimale Friktion zwischen der Spitze 31 und dem Führungsdraht 40 besteht.

Es ist auch möglich, dass das Funktionselement 10 mehrere Haltelemente 30 umfasst, die axialverschieblich mit dem Führungsdraht 40 verbunden sind, wie in den Fig. 3a und 3b dargestellt. Bei dem Ausführungsbeispiel gemäß Fig. 3a und 3b weisen die Halteelemente 30 jeweils eine V-förmige Kontur mit einer als Schlaufe 31b ausgebildeten Spitze 31 auf. Die Schlaufe 31b der einzelnen Halteelemente 30 umschlingt jeweils den Führungsdraht 40. Wie in Fig. 3a dargestellt, sind mehrere Halteelemente 30 in längsaxialer Richtung des Funktionselements 10 hintereinander angeordnet, insbesondere fluchtend hintereinander angeordnet. Die Halteelemente 30 sind also in längsaxialer Richtung des Funktionselements 10 beabstandet. Zusätzlich können, wie in Fig. 3b dargestellt, auf derselben Querschnittshöhe mehrere Halteelemente 30 über den Umfang der Gitterstruktur 20 verteilt angeordnet sein. Dabei können jeweils zwei Halteelemente 30 gegenüber angeordnet sein. Die Halteelemente 30 können in Kreuzform angeordnet sein, so dass die Halteelemente 30 jeweils um 90 Grad versetzt über den Umfang verteilt angeordnet sind. Hieraus ergibt sich, dass auf jeweils einer Querschnittshöhe vier Halteelemente 30 angeordnet sind. Eine andere Anzahl von Halteelementen auf einer Querschnittshöhe, die dann jeweils um einen anderen Umfang auf den Umfang versetzt verteilt angeordnet sind, ist möglich. Beispielsweise können 3 Halteelemente 30 oder mehr als 4 Halteelemente 30 vorgesehen sein. Alternativ können die Halteelemente 3 auch helixförmig bzw. spiralförmig angeordnet sein. Die in längsaxialer Richtung aufeinanderfolgenden Halteelemente 30 können also jeweils um einen Winkel um die Längsachse des Funktionselements 10 versetzt angeordnet sein, so dass sich insgesamt eine spiralförmige Anordnung ausbildet. Im Allgemeinen können die Halteelemente 30 unterschiedliche Längen aufweisen, um sicherzustellen, dass der Führungsdraht 40 im expandierten Zustand des Funktionselements 10 längsaxial, insbesondere geradlinig, in dem Funktionselement 10 angeordnet ist. Dies gilt für alle Ausführungsbeispiele.

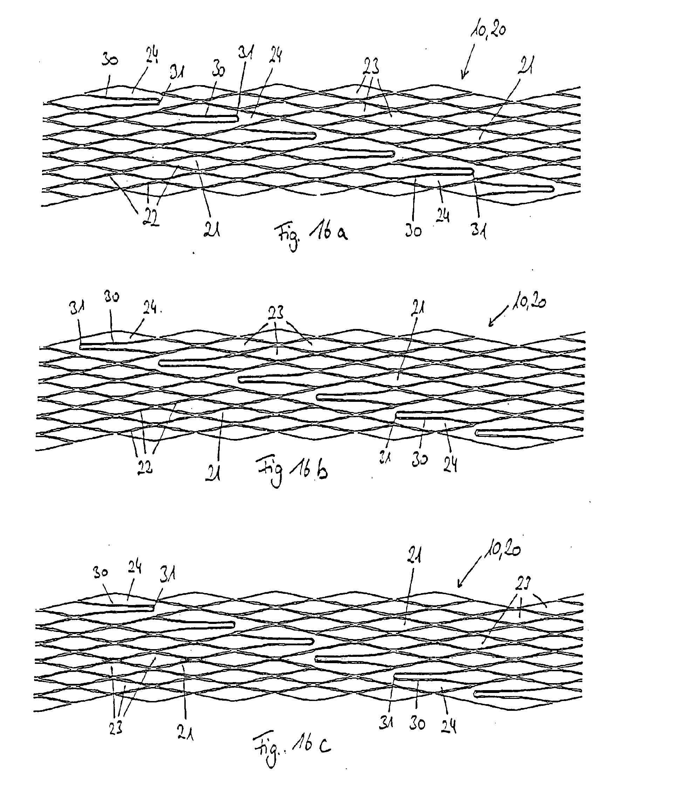

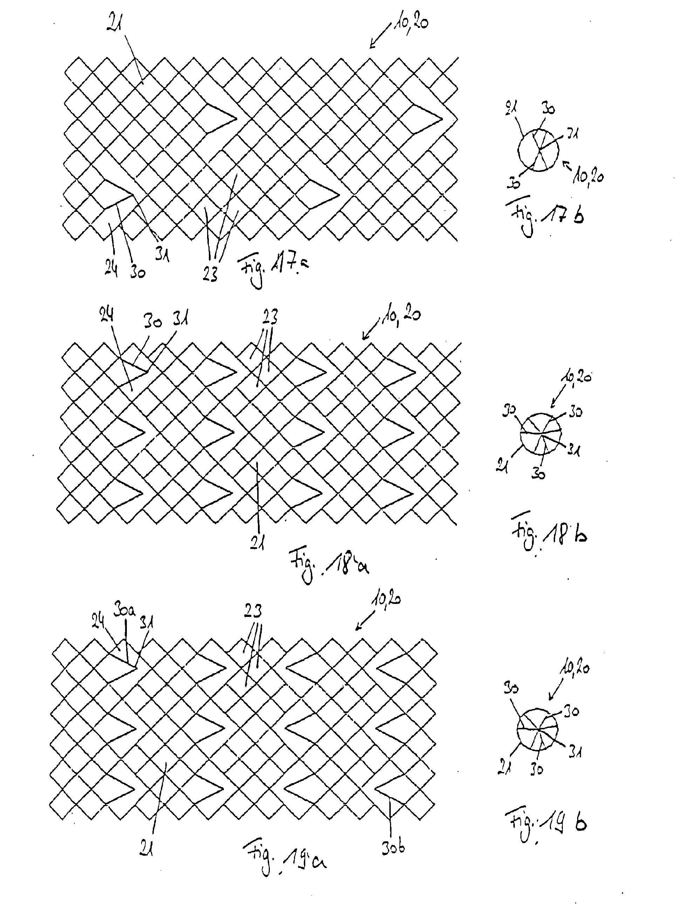

Eine Gitterstruktur 20 mit Halteelementen 30, die unterschiedliche Längen aufweisen, ist beispielsweise in Fig. 16 dargestellt. Dabei weist ein erstes Halteelement 30a eine erste Länge L1 und ein zweites Halteelement 30b eine zweite Länge L2 auf. Die Ermittlung der Längen L1, L2 erfolgt im Wesentlichen entlang der Längsachse des Funktionselements 10, wenn die Halteelemente 30 längsaxial ausgerichtet sind. Im Allgemeinen können die Halteelemente 30 längsaxial, also entlang der Längsachse des Funktionselements 10, und/oder in Umfangsrichtung des Funktionselements 10 angeordnet sein. Entsprechend erfolgt die Ermittlung der Länge des Halteelements 30 in Umfangsrichtung des Funktionselements 10, wenn das Halteelement 30 in Umfangsrichtung ausgerichtet ist. Wie in Fig. 16 gut zu erkennen, sind die Speichen 33a, 33b des ersten Halteelements 30a und des zweiten Halteelements 30b jeweils in derselben Umfangsebene des Funktionselements 10 bzw. der Gitterstruktur 20 mit den Stegen 22 der Gitterstruktur 20 verbunden. Insbesondere sind die Halteelemente 30a, 30b jeweils mit Knotenpunkten 25 der Gitterstruktur 20 verbunden, die in derselben Querschnittsebene bzw. derselben Umfangslinie des Funktionselements 10 angeordnet sind. Aufgrund der unterschiedlichen Längen L1, L2 der Halteelemente 30a, 30b sind die Spitzen 31 der Halteelemente 30a, 30b hingegen in unterschiedlichen Querschnittsebenen bzw. Umfangslinien des Funktionselements 10 angeordnet. Das hat zur Folge, dass die Spitzen 31 der Halteelemente 30a, 30b im expandierten Zustand des Funktionselements 10 axial zueinander versetzt mit dem Führungsdraht 40 verbunden bzw. an den Führungsdraht 40 angeordnet sind, wie in Fig. 17a dargestellt. Fig. 17b zeigt die Anordnung der Halteelemente 30a, 30b in einer Querschnittsdarstellung. Darin ist zu erkennen, dass die Halteelemente 30a, 30b im expandierten Zustand des Funktionselements 10 queraxial gegenüberliegend angeordnet sind. Durch die versetzte Anordnung der Spitzen 31 der Halteelemente 30a, 30b wird sichergestellt, dass der Führungsdraht 40 im Wesentlichen achsengleich mit dem Funktionselement 10 ausgerichtet ist.

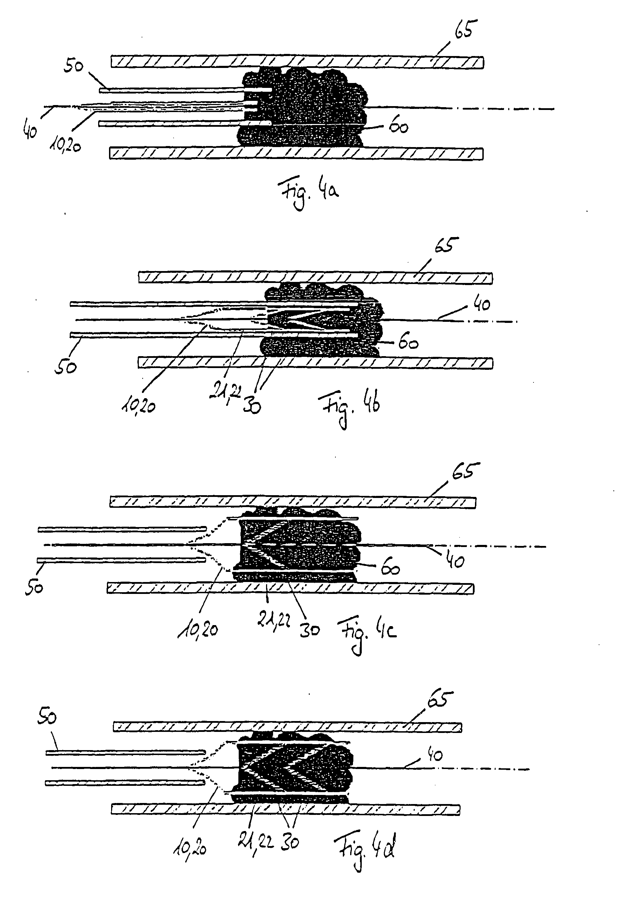

Die Funktionsweise der medizinischen Vorrichtung ist in den Fig. 4a bis 4d beispielhaft dargestellt. In den Fig. 4a bis 4d ist ein Blutgefäß 65 gezeigt, in dem ein Konkrement 60 angeordnet ist. Das Konkrement 60 kann beispielsweise einen Thrombus, also ein Blutgerinnsel, umfassen. Das Blutgerinnsel bzw. das Konkrement 60 verschließt das Blutgefäß 65, so dass eine Nährstoffversorgung stromabwärts gelegener Gewebeareale unterbrochen ist. Zur Entfernung des Konkrements 60 wird zunächst ein Katheter 50 an den Behandlungsort, also in den Bereich des Konkrements 60, geführt. Das distale Ende des Katheters 50 kann den Thrombus bzw. das Konkrement 60 berühren. Durch den in dem Katheter 50 ausgebildeten Hohlkanal wird das Funktionselement 10 mit dem Führungsdraht 40 nach distal bis zu dem Konkrement 60 geschoben. Das Funktionselement 10 ist dabei mit dem Führungsdraht 40 verbunden. Innerhalb des Katheters 50 ist das Funktionselement 10 komprimiert bzw. weist den komprimierten Zustand auf (Fig. 4a). In einem nächsten Schritt wird der Katheter 50 mit dem Funktionselement 10 und dem Führungsdraht 40 nach distal verschoben und durchdringt dabei das Konkrement 60 (Fig. 4b). Anschließend wird der Katheter 50 in proximale Richtung zurückgezogen, wodurch der äußere Zwang, der das Funktionselement 10 im komprimierten Zustand gehalten hat, aufgegeben wird. Dadurch expandiert das Funktionselement 10. Konkret weist das Funktionselement 10, insbesondere die Gitterstruktur 20, eine Radialkraft auf, die die Expansion der Gitterstruktur 290 bzw. des Funktionselements 10 bewirkt. Dabei durchdringt der Schneidbereich 21 der Gitterstruktur 20 das Konkrement 60. Mit anderen Worten schneiden die Stege 22 des Schneidbereichs 21 der Gitterstruktur 20 in das Konkrement 60 ein. Dabei weitet sich das Funktionselement 10 in radialer Richtung auf. Das Durchdringen des Konkrements 60 mit den Stegen 22 bzw. dem Schneidbereich 21 erfolgt also in radialer Richtung. Die mit dem Führungsdraht verbundenen Halteelemente 30 werden bei der Expansion des Funktionselements in radialer Richtung nach innen ausgelenkt und bilden somit eine Verankerung für das Konkrement 60 (Fig. 4c). Sobald das Funktionselement 10 vollständig expandiert ist, kann das Konkrement 60 durch eine proximale Bewegung des Führungsdrahts 40, der fest mit dem Funktionselement 10 verbunden ist, aus dem Blutgefäß 65 entfernt werden (Fig. 4d).

Vorteilhaft ist vorgesehen, dass das Funktionselement bei vollständiger Expansion einen Querschnittsdurchmesser aufweist, der kleiner als der Innendurchmesser des Blutgefäßes 65 ist. Dadurch wird vermieden, dass das Funktionselement 10 die Gefäßwand des Blutgefäßes 65 verletzt, insbesondere beim Entfernen des Konkrements 60 durch Zurückziehen des Funktionselements 10.

Durch die Verbindung der Halteelemente 30 mit dem Führungsdraht 40 wird sichergestellt, dass die Halteelemente 30 das Lumen des Funktionselements 10 von der Gitterstruktur 20, mit der die Halteelemente 30 verbunden sind, bis zur Mittellinie der Gitterstruktur 20 überspannen. Damit wird erreicht, dass das Funktionselement 10 einen großen wirksamen Querschnitt für die Verankerung des Funktionselements 10 mit dem Konkrement aufweist.

Ferner ist es möglich, das Funktionselement bis auf einen Durchmesser aufzudehnen, der kleiner als der Gefäßdurchmesser ist, um beim Entfernen des Funktionselements einen Abstand zur Gefäßwand einzuhalten, so dass diese bei der Behandlung geschont wird. Insofern erfüllt die Radialkraft zwei konkurrierende Anforderungen, nämlich einerseits eine ausreichende Schneidkraft aufzubringen und andererseits einen Abstand der Gitterstruktur zur Gefäßwand einzuhalten.

Auch wenn die Möglichkeit der schonenden Behandlung durch Vermeidung der Berührung der Gefäßwand ein Vorteil des Thrombektomie devices ist, wird nicht ausgeschlossen, dass die Gitterstruktur bspw. zur Behandlung schwer lösbarer Konkremente so ausgelegt werden kann, dass diese im expandiertem Zustand in Kontakt mit der Gefäßwand kommt oder sogar eine Radialkraft auf die Gefäßwand ausübt derart, dass die Gefäßwand gedehnt wird. Dadurch erhöht sich die Kraft, mit der das Konkrement beaufschlagt wird.

Zur Behandlung von Gefäßen bspw. mit abnehmendem Durchmesser kann die Gitterstruktur in Längsrichtung unterschiedliche Durchmesser aufweisen. Dabei kann ein Bereich der Gitterstruktur kleiner und ein anderer Bereich gleich groß oder größer als der Durchmesser des Gefäßes sein. Die Gitterstruktur kann so ausgelegt sein, dass der Durchmesser der Gitterstruktur distal kleiner ist als proximal. Der Durchmesser nimmt also in distaler Richtung ab. Damit wird vermieden, dass die Gitterstruktur ein Gefäß mit distal abnehmendem Durchmesser bzw. distale Gefäße mit kleinerem Durchmesser nicht verletzt. Andererseits bietet der proximal vergrößerte Durchmesser der Gitterstruktur einen bessere Verankerung der Gitterstruktur im Konkrement im Bereich des Gefäßes mit größerem Durchmesser.

Es ist auch möglich, dass der Durchmesser der Gitterstruktur proximal kleiner ist als distal. Der Durchmesser wird also in distaler Richtung größer. Dadurch wird eine größere Radialkraft auf die Gefäßwand im distalen Gefäßbereich ausgeübt. Der distale Bereich der Gitterstruktur funktioniert somit als Filter, der verhindert, dass sich lösende Partikel fortgeschwemmt werden und distal gelegene Gefäßabschnitte verschließen.

Der Durchmesser des größeren Bereichs der Gitterstruktur (distal oder proximal) kann z.B. mindestens 20%, insbesondere mindestens 40%, insbesondere mindestens 60%, insbesondere mindestens 80%, insbesondere mindestens 100% größer sein als der Durchmesser des kleineren Bereichs der Gitterstruktur.

Für alle Ausführungsbeispiele gilt, dass das Halteelement 30 vorzugsweise eine kleinere mechanische Widerstandskraft bzw. eine höhere Flexibilität aufweist als die Stege 22 des Schneidbereichs 21. Dies kann beispielsweise dadurch erreicht werden, dass die Breite der Speichen 33a, 33b kleiner ist als die Breite der Stege 22. In Fig. 5 ist beispielhaft die Speichenbreite S1 und die Stegbreite S2 dargestellt. Es ist gut zu erkennen, dass die Speichenbreite S1 kleiner als die Stegbreite S2 ist. Die Breite der Speichen 33a, 33b, also die Speichenbreite S1, kann zwischen 10 µm und 50 µm betragen. Die Breite der Stege 22, also die Stegbreite S2, kann hingegen wenigstens 20 µm und höchstens 100 µm betragen.

In Fig. 5 ist ferner zu erkennen, dass das Halteelement 30 innerhalb einer Zelle 23 der Gitterstruktur angeordnet ist. Die Speichen 33a, 33b sind dabei direkt mit jeweils einem Steg 22 der Gitterstruktur 20 verbunden. Die Form des Halteelements 30 entspricht im Übrigen der Form des zweiten Halteelements 30 gemäß Fig. 1, wobei die Spitze 31 eine Schlaufe 31b bildet.

Das Funktionselement 10 weist im Allgemeinen sowohl im expandierten als auch im komprimierten Zustand eine zylindrische Grundform auf. Die rotationssymmetrische Gitterstruktur 20 ist somit sowohl im expandierten als auch im komprimierten Zustand erkennbar.

Bei der Expansion des Funktionselements 10 innerhalb des Konkrements 60 wird durch die unterschiedlichen mechanischen Eigenschaften bzw. Flexibilitäten oder Elastizitäten des Halteelements 30 gegenüber den Stegen 22 des Schneidbereichs 21 unterschiedliche Expansionsgrade des Funktionselements 10 erreicht. Die unterschiedlichen mechanischen Eigenschaften bzw. unterschiedlichen mechanischen Steifigkeiten führen zu unterschiedlichen lokalen Radialkräften des Funktionselements 10. Konkret weist der Schneidbereich 21 eine höhere Radialkraft bzw. Expansionskraft auf als das Halteelement 30. Da die Größe der Radialkraft das Durchdringungspotential des Funktionselements beim Expandieren innerhalb des Konkrements 60 bestimmt, schneiden die Stege 22 des Schneidbereichs 21 in das Konkrementmaterial ein, wogegen das Halteelement 30 radial nach innen ausgelenkt wird. Die Auslenkung des Halteelements 30 erfolgt also nicht nur durch die Verbindung mit dem Führungsdraht 40, sondern auch durch den äußeren Druck des Konkrements 60 auf das Halteelement 30.

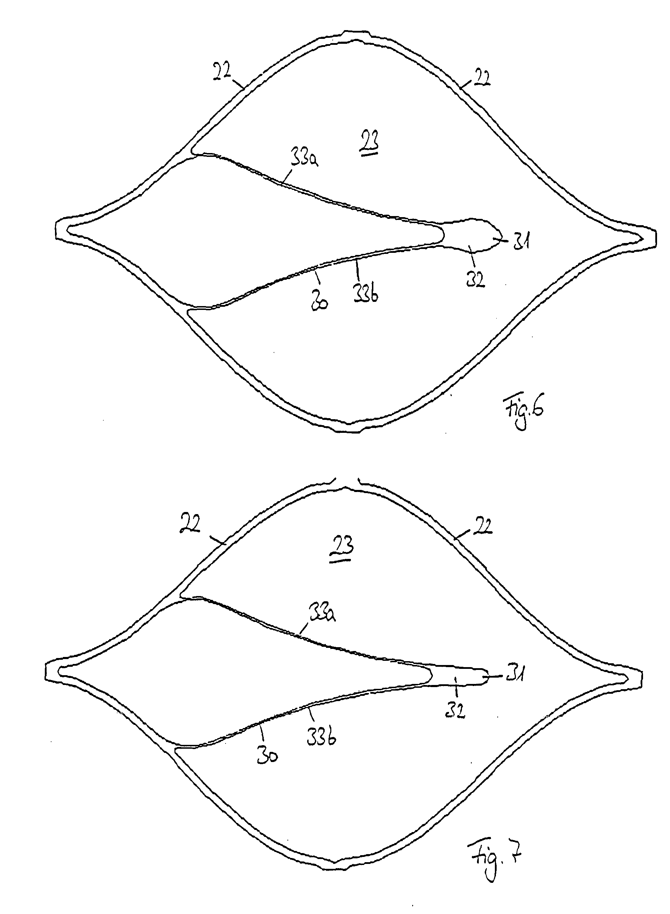

Dieser Effekt kann konstruktiv vorteilhaft genutzt werden, um die radiale Auslenkung des Halteelements 30 zu unterstützen. Dazu ist in den Ausführungsbeispielen gemäß Fig. 6 bis 8 jeweils vorgesehen, eine Berührungsfläche des Halteelements 10 mit dem Konkrement 60 zu vergrößern. Bei dem Ausführungsbeispiel gemäß Fig. 6 weist die Spitze 31 des Halteelements 30 beispielsweise eine vergrößerte Kontaktfläche 32 auf. Insbesondere ist die Spitze 31 des Halteelements 30 löffelartig ausgebildet. Eine ähnlich ausgebildete Kontaktfläche 32 der Spitze 31 ist in Fig. 7 dargestellt. Die Ausführungsbeispiele gemäß Fig. 6 und 7 unterscheiden sich darin, dass die Kontaktfläche 32 gemäß Fig. 6 seitlich ausgewölbt ist, um eine löffelartige Kontur zu bilden. Die Kontaktfläche 32 gemäß Fig. 7 weist hingegen gerade Seitenkanten auf, so dass sich vielmehr eine spatelartige Kontur bildet.

Eine weitere Variante zur Erhöhung der Berührungsfläche des Halteelements 30 mit dem Konkrement 60 ist in Fig. 8 dargestellt. Dabei weisen die Speichen 33a, 33b jeweils wenigstens eine flügelartige Verbreiterung auf, die eine Kontaktfläche 32 bildet. Konkret sind bei dem Ausführungsbeispiel gemäß Fig. 8 jeweils zwei flügelartige Verbreiterungen an jeder Speicher 33a, 33b vorgesehen. Vorzugsweise sind die flügelartigen Verbreiterungen bzw. Kontaktflächen 32 an einer Außenkontur des V-förmigen Haltelements angeordnet.

In Fig. 9 ist ein weiteres Ausführungsbeispiel dargestellt, wobei die Spitzen 31 der Halteelemente 30 jeweils eine vergrößerte Kontaktfläche 32 aufweisen. Konkret ist bei dem Ausführungsbeispiel gemäß Fig. 9 vorgesehen, in einer Zelle 23 mehr als ein Halteelement 30, insbesondere zwei Halteelemente 30, anzuordnen. Die Halteelemente 30 weisen jeweils erste und zweite Speichen 33a, 33b auf. Die ersten Speichen 33a sind dabei mit einem ersten Steg 22a und die zweiten Speichen 33b mit einem zweiten Steg 22b verbunden. Dabei sind die Halteelemente 30 derart angeordnet, dass die Spitzen 31 in längsaxialer Richtung des Funktionselements 10 bzw. der Gitterstruktur 20 einander nachgeordnet sind. Es ist möglich, dass alle Halteelemente 30 einer Zelle 23 mit dem Führungsdraht 40 verbindbar sind. Alternativ kann ein Halteelement 30, insbesondere das erste Halteelement 30a, das größer als das zweite Halteelement 30b ist, mit dem Führungsdraht 40 verbunden, und das anderen Halteelement 30, insbesondere das zweite Halteelement 30b, frei angeordnet sein. Bei dem freien Halteelement 30, insbesondere dem zweiten Halteelement 30b, erfolgt die radial nach innen gerichtete Auslenkung bei der Expansion des Funktionselements 10 nicht über den Führungsdraht 40, sondern im Wesentlichen selbsttätig durch das Zusammenwirken des freien Halteelements 30 mit dem Konkrement 60.

Im Allgemeinen kann die Gitterstruktur 20 des Funktionselements 10 Halteelemente 30 aufweisen, die mit dem Führungsdraht 40 verbunden sind und weitere, freie Halteelemente 30 umfassen, die lose angeordnet sind.

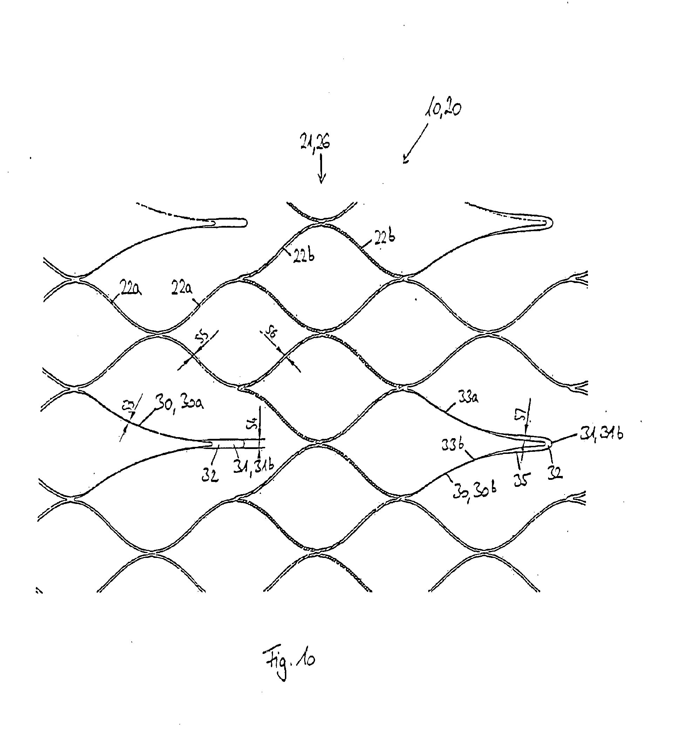

Die bereichsweise unterschiedlichen mechanischen Eigenschaften bzw. mechanischen Steifigkeiten der Gitterstruktur 20 können durch unterschiedliche geometrische Ausgestaltungen einzelner Elemente der Gitterstruktur 20 erreicht werden. Vorteilhafterweise können unterschiedliche Breiten der Stege 22 bzw. der Speichen 33a, 33b des Halteelements 30 vorgesehen sein. In Fig. 10 sind unterschiedliche Breiten verschiedener Elemente der Gitterstruktur 20 dargestellt. Beispielsweise weisen die Speichen 33a, 33b des Halteelements 30 eine Speichenbreite S3 auf. Die Gitterstruktur 20 weist ferner einen Schneidbereich 21 auf, der aus mehreren ersten Stegen 22a mit einer ersten Stegbreite S5 und mehreren zweiten Stegen 22b mit einer zweiten Stegbreite S6 gebildet ist. Vorzugsweise ist die Speichenbreite S3 kleiner als die erste Stegbreite S5. Die erste Stegbreite S5 ist vorzugsweise kleiner als die zweite Stegbreite S6. Auf diese Weise bilden die zweiten Stege 22 einen Teil des Schneidbereichs 21, der eine höhere Durchdringungskraft aufweist, als der Teil des Schneidbereichs 21, der durch die ersten Stege 22a gebildet ist. Vorzugsweise begrenzen die zweiten Stege 22b Zellen 23, die einen Axialabschnitt bzw. einen Zellenring 26 der Gitterstruktur 20 bilden. Die Speichenbreite S3 der Speichen 33a, 33b des Halteelements 30 beträgt vorzugsweise wenigstens 10 µm und/oder höchstens 50 µm. Für die Stegbreiten S5, S6 ist ein Wertebereich von wenigstens 20 µm bis höchstens 100 µm vorteilhaft, wobei die erste Stegbreite S5 kleiner als die zweite Stegbreite S6 ist.

Bei dem Ausführungsbeispiel gemäß Fig. 10 weist die Spitze 31 eines ersten Halteelements 30 eine vergrößerte, insbesondere spateiförmige, Kontaktfläche 32 auf. Die Kontaktfläche 32 bzw. die Spitze 31 umfasst eine Spitzenbreite S4, die vorzugsweise wenigstens 50 µm und/oder höchstens 500 µm beträgt. Ein zweites Halteelement 30b weist ebenfalls eine Spitze 31 mit einer vergrößerten Kontaktfläche 32 auf. Die Spitze 31 bildet eine Schlaufe 31b. Ferner umfasst das zweite Halteelement 30b einen Übergangsbereich 35, der zwischen jeweils einer Speiche 33a, 33b und der Spitze 31 ausgebildet ist. Der Übergangsbereich zeichnet sich durch eine verbreiterte Kontur der Speiche 33a, 33b aus. Konkret kann der Übergangsbereich 35 eine Übergangsbreite S7 aufweisen, die wenigstens 50 µm beträgt. Die Übergangsbreite S7 kann höchstens 200 µm betragen.

Die Funktionsweise der medizinischen Vorrichtung wird insbesondere durch die Geometrie der Gitterstruktur 20, konkret durch die Größe der Zellen 23, bestimmt. Beispielsweise weisen vergleichsweise große Zellen 23, die durch Stege 22 begrenzt sind, die einen großen Stegquerschnitt umfassen, eine erhöhte Radialkraft und somit eine kleinere Flexibilität auf. Zellen 23 mit Stegen 22, die einen großen Stegquerschnitt aufweisen, bilden vorzugsweise einen Schneidbereich 21, da derartig geformte Zellen 23 bzw. Stege 22 ein hohes Durchdringungspotential aufweisen. Die Stege 22 des Schneidbereichs 21, die einen großen Stegquerschnitt umfassen, ermöglichen also ein verbessertes Einschneiden in ein Konkrement 60. Kleinere Zellen 23, deren Stege 22 einen kleineren Stegquerschnitt aufweisen, umfassen eine vergleichsweise geringe Radialkraft und somit eine höhere Flexibilität. Derartig gestaltete Zellen 23 bzw. Stege 22 können daher eine Filter- bzw. Rückhaltewirkung auf das Konkrement 60 bzw. sich ablösende Konkrementpartikel ausüben.

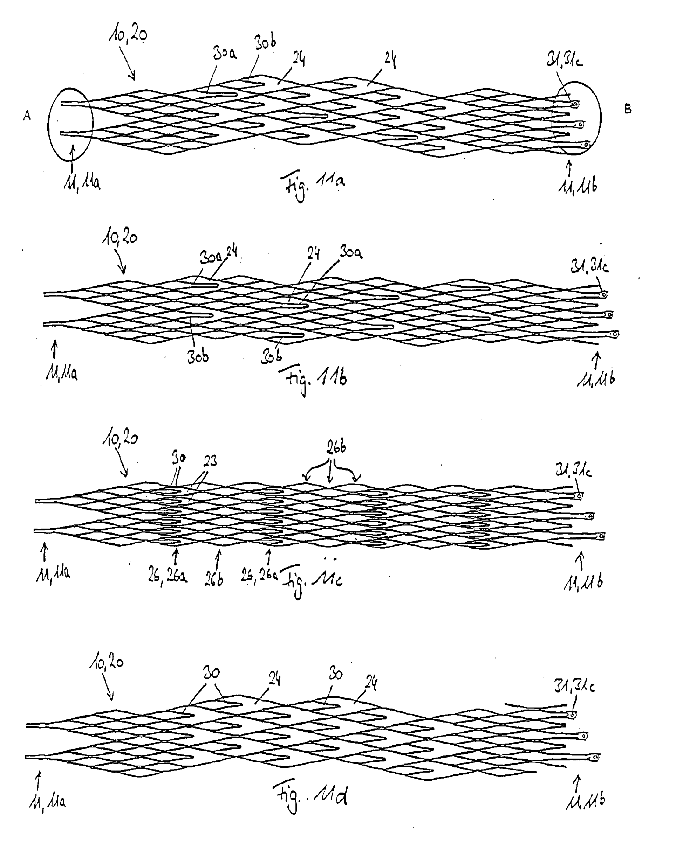

In den Fig. 11a bis 11d sind mehrere Ausführungsbeispiele des Funktionselements 10 dargestellt, wobei das Funktionselement 10 eine Gitterstruktur 20 mit mehreren Halteelementen 30 umfasst. Die Halteelemente 30 sind bei den Ausführungsbeispielen gemäß Fig. 11a, 11b und 11d jeweils in Maschen 24 der Gitterstruktur 20 angeordnet. Bei dem Ausführungsbeispiel gemäß Fig. 11b sind die Halteelemente 30 hingegen in den Zellen 23 der Gitterstruktur 20 angeordnet.

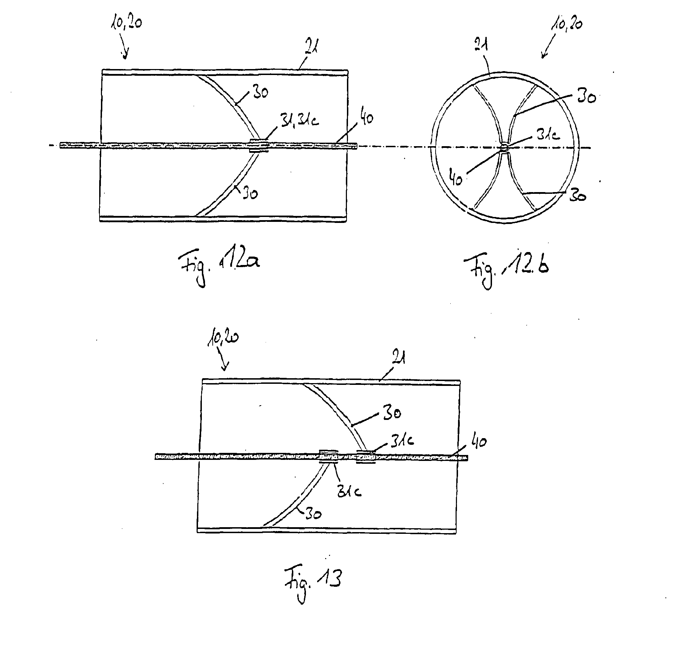

Das Funktionselement 10 weist zwei axiale Enden 11, insbesondere ein proximales Ende 11a und ein distales Ende 11b auf. Das proximale Ende 11a kann mit dem Führungsdraht 40 fest verbunden sein. Insbesondere weist das Funktionselement 10 gemäß Fig. 13a einen proximalen Endbereich A auf, der mit dem Führungsdraht 40 fest verbunden ist (nicht dargestellt). Die feste Verbindung zwischen dem proximalen Endbereich A bzw. dem proximalen Ende 11a des Funktionselements 10 und dem Führungsdraht 40 kann beispielsweise durch Kleben, Schweißen, Löten oder Crimpen erfolgen. Ferner umfasst das Funktionselement 10 ein distales Ende 11b bzw. einen distalen Endbereich B, der durch Halteelemente 30 gebildet ist. Die Halteelemente 30 weisen jeweils eine Spitze 31 auf, die als Schlaufe 31b, Öse 31a oder Gleithülse 31c ausgebildet sein kann. Bei dem Ausführungsbeispiel gemäß Fig. 11a bis 11d weisen die Halteelemente 30 am distalen Ende 11b des Funktionselements 10 jeweils eine Gleithülse 31c auf. Die Gleithülsen 31c sind längsaxial verschiebbar mit dem Führungsdraht verbunden. Konkret kann der Führungsdraht 40 durch die Gleithülsen 31c hindurchgeführt sein. Dazu ist vorteilhaft vorgesehen und in den Fig. 11a bis 11d dargestellt, dass die Halteelemente 30 am distalen Ende 11b des Funktionselements bzw. im distalen Endbereich B unterschiedliche Längen aufweisen, so dass die Gleithülsen 31c in axialer Richtung nachgeordnet auf dem Führungsdraht 40 angeordnet sind. Über den Umfang des Funktionselements 10 erstrecken sich am distalen Ende 11b des Funktionselements 10 mehrere Halteelemente 30, die mit dem Führungsdraht 40 verschiebbar verbunden sind. Dadurch wird im expandierten Zustand des Funktionselements 10 am distalen Ende 11b des Funktionselements 10 bzw. im distalen Endbereich B ein netzartiger Verschluss des Funktionselements 10 bzw. der Gitterstruktur 20 gebildet. Das Funktionselement 10 weist somit einen geschlossenen distalen Endbereich B auf. Es ist im Übrigen auch möglich, dass die Spitzen 31 der Halteelemente 30 am distalen Ende 11b des Funktionselements 10 zur Aufnahme von Röntgenmarkierungen angepasst und vorgesehen sind.

Die Gitterstruktur 20 bei dem Ausführungsbeispiel gemäß Fig. 11a weist mehrere erste Halteelemente 30a und mehrere zweite Haltelemente 30b auf, wobei die ersten Halteelemente 30a eine größere Länge L1, L2 aufweisen, als die zweiten Halteelemente 30b. Dabei sind die zweiten Halteelemente 30b jeweils in einer Umfangslinie bzw. einem Axialabschnitt der Gitterstruktur 20 angeordnet. Das bedeutet, dass die zweiten Halteelemente 30b jeweils ringförmig in einem Axialabschnitt des Funktionselements 10 angeordnet sind. Die ersten Halteelemente 30a sind hingegen zueinander in axialer Richtung und in Umfangsrichtung versetzt angeordnet. Konkret sind die ersten Halteelemente 30a im Wesentlichen helixartig bzw. spiralförmig in der Gitterstruktur 20 angeordnet. Vorzugsweise sind die ersten Halteelemente 30a der Gitterstruktur 20 mit dem Führungsdraht 40 verbunden. Die zweiten Halteelemente 30b können ebenfalls mit dem Führungsdraht 40 verbunden sein. Vorzugsweise bilden die zweiten Halteelemente 30b bei dem Ausführungsbeispiel gemäß Fig. 11a freie bzw. lose angeordnete Halteelemente 30.

Das Ausführungsbeispiel gemäß Fig. 11b unterscheidet sich von dem Ausführungsbeispiel gemäß Fig. 11a durch die Anordnung der Halteelemente 30. Konkret ist bei dem Ausführungsbeispiel gemäß Fig. 11b vorgesehen, mehrere erste Halteelemente 30a spiralförmig bzw. helixförmig entlang des Funktionselements 10 anzuordnen. Ferner sind mehrere zweite Halteelemente 30b vorgesehen, die ebenfalls helixförmig bzw. spiralförmig entlang des Funktionselements 10 angeordnet sind, wobei jeweils ein erstes Halteelement 30a und ein zweites Halteelement 30b in demselben Axialabschnitt des Funktionselement 10 angeordnet sind. Das erste Halteelement 30a kann jeweils eine größere Länge L1, L2 aufweisen, also das zweite Halteelement 30b. Es ist auch möglich, dass das erste Halteelement 30a und das zweite Halteelement 30b dieselbe Länge L1, L2 aufweisen. Die ersten Halteelemente 30a und/oder die zweiten Halteelemente 30b können mit dem Führungsdraht 40 verbunden sein.

Bei dem Ausführungsbeispiel gemäß Fig. 11c sind mehrere Halteelemente vorgesehen, die im Wesentlichen dieselbe Länge aufweisen. Jeweils ein Halteelement 30 ist in einer Zelle 23 der Gitterstruktur 20 des Funktionselements 10 angeordnet. Vorzugsweise sind in jeder Zelle 23 eines Zellenrings 26 bzw. eines Ringsegments jeweils ein Halteelement 30 angeordnet. Insgesamt weist das Funktionselement 10 mehrere erste Zellenringe 26a und mehrere zweite Zellenringe 26b auf, wobei in den ersten Zellenringen 26a Halteelemente 30 angeordnet sind. Die Zellen 23 der zweiten Zellenringe 26b sind frei bzw. weisen keine Halteelemente 30 auf. Vorzugsweise sind jeweils drei zweite Zellenringe 26b zwischen zwei ersten Zellenringen 26a angeordnet. Eine andere Anzahl von zweiten Zellenringen 26b zwischen zwei ersten Zellenringen 26a ist möglich.

In Fig. 11d ist ein weiteres Ausführungsbeispiel dargestellt, das einen ähnlichen Aufbau der Gitterstruktur 20 zeigt, wie das Ausführungsbeispiel gemäß Fig. 11a. Der Unterschied zu dem Ausführungsbeispiel gemäß Fig. 11a besteht darin, dass bei dem Ausführungsbeispiel gemäß Fig. 11d die Halteelemente 30, also die ersten Halteelemente 30a und die zweiten Halteelemente 30b, dieselbe Länge L1, L2 aufweisen.

Im Hinblick auf die axialverschiebbare Verbindung zwischen dem Funktionselement 10 und dem Führungsdraht 40 ist in den Fig. 12a bis 13 eine alternative Verbindungsmöglichkeit dargestellt. Alternativ oder zusätzlich zu einer Öse 31a oder einer Schlaufe 31d kann die Spitze 31 eine Gleithülse 31c umfassen. Die Gleithülse 31c kann mit der Spitze 31 bzw. allgemein mit dem Halteelement 30 durch Schweißen, Kleben, Löten oder Crimpen verbunden sein. Die Gleithülse 31c kann axialverschieblich auf dem Führungsdraht 40 angeordnet sein. Es ist möglich, dass mehrere Halteelemente 30 mit einer Gleithülse 31c verbunden sind, wie in den Fig. 12a und 12b dargestellt. Alternativ kann jedem Halteelement 30 eine einzige Gleithülse 31c zugeordnet sein, wie in Fig. 13 gezeigt. Die Hülsen 31c unterschiedlicher Halteelemente 30 sind vorzugsweise in axialer Richtung des Führungsdrahts 40 einander nachgeordnet. Im Allgemeinen kann die Gleithülse 31c ein Metall, einen Kunststoff und/oder ein röntgensichtbares Material umfassen.

In den Fig. 16a bis 16c ist gezeigt, dass die Halteelemente 30 in unterschiedlichen Richtungen orientiert sein können. Bei dem Ausführungsbeispiel gemäß Fig. 16a sind beispielsweise mehrere Halteelemente 30 vorgesehen, die in Maschen 24 der Gitterstruktur 20 angeordnet sind. Die Maschen 24 bzw. die Halteelemente 30 erstrecken sich spiralförmig entlang des Funktionselements 10 bzw. der Gitterstruktur 20. Die Halteelemente 30 weisen dieselbe Richtungsorientierung auf. Konkret sind die Halteelemente 30 bei dem Ausführungsbeispiel gemäß Fig. 16a in distaler Richtung ausgerichtet. Das bedeutet, dass die Spitze 31 dies Halteelements 30 in Richtung eines distalen Endes 11b des Funktionselements 10 zeigt. Im Unterschied dazu ist bei dem Ausführungsbeispiel gemäß Fig. 16b vorgesehen, dass die Halteelemente 30 in proximaler Richtung orientiert sind. Die Spitzen 31 der Halteelemente 30 gemäß Fig. 16b zeigen also in Richtung eines proximalen Endes 11a des Funktionselements 10. Eine Kombination von proximal ausgerichteten Halteelementen 30 und distal ausgerichteten Halteelementen 30 ist ebenfalls möglich, wie in Fig. 16c dargestellt.

Die Fig. 17a bis 19b zeigen Ausführungsbeispiele der medizinischen Vorrichtung, wobei die Halteelemente 30 jeweils in Maschen 24 der Gitterstruktur 20 angeordnet sind. Die Fig. 17a bis 19b verdeutlichen die unterschiedlichen Anordnungsvarianten der Halteelemente 30. Beispielsweise ist in Fig. 17a dargestellt, dass mehrere Halteelemente 30 sowohl in axialer Richtung, als auch in Umfangsrichtung der Gitterstruktur 20 versetzt angeordnet sein können. Dabei ist in jedem axialen Abschnitt der Gitterstruktur 20 bzw. des Funktionselements 10 ein einziges Halteelement 30 angeordnet. Die in Umfangsrichtung zueinander versetzten Halteelemente sind in der Querschnittsansicht gemäß Fig. 17b diametral gegenüberliegend angeordnet. Die V-förmigen Halteelemente 30 bilden somit in der Querschnittsansicht gemäß Fig. 17b eine X-förmige Struktur. Die Halteelemente 30 gemäß Fig. 17a bis 19b sind jeweils mit dem Führungsdraht 40 verbunden (nicht dargestellt).

Bei dem Ausführungsbeispiel gemäß Fig. 18a und 18b sind jeweils drei bzw. allgemein mehrere Halteelemente 30 in einem Axialabschnitt des Funktionselements 10 angeordnet. Die Halteelemente 30 fluchten sowohl in axialer Richtung, als auch in Umfangsrichtung des Funktionselements 10. Dabei weisen die Halteelemente 30 dieselbe Richtungsorientierung auf. In der Querschnittsansicht gemäß Fig. 18b ist zu erkennen, dass die Halteelemente 30 jeweils in einem Winkel von etwa 120 Grad auf dem Umfang der Gitterstruktur 20 angeordnet sind. In der Querschnittsansicht bildet sich somit eine sternförmige Kontur der Halteelemente 30.

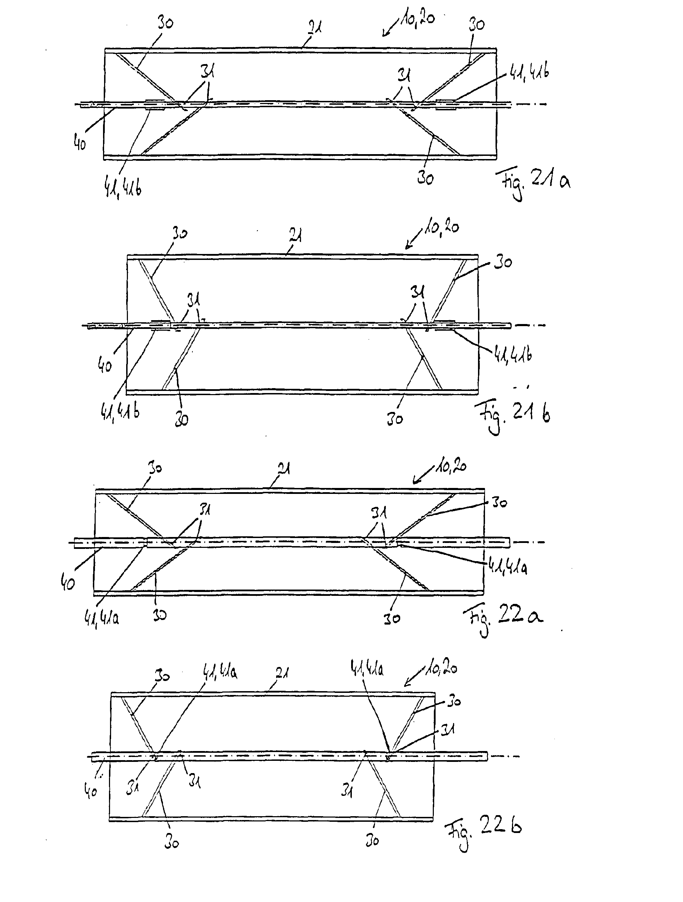

Eine ähnliche Struktur weist das Ausführungsbeispiel gemäß Fig. 19a, 19b auf. Konkret ist in der Querschnittsansicht gemäß Fig. 19b ebenfalls eine sternförmige Kontur der Halteelemente 30 zu erkennen. Im Unterschied zu dem Ausführungs beispiel gemäß Fig. 18a, 18b sind bei dem Ausführungsbeispiel gemäß Fig. 21a, 21b mehrere erste Halteelemente 30a und mehrere zweite Halteelemente 30b vorgesehen, wobei die zweiten Halteelemente 30b eine andere Richtungsorientierung aufweisen, als die ersten Halteelemente 30a. Konkret sind die zweiten Halteelemente 30b gegenüber den ersten Halteelementen 30a gegenläufig bzw. spiegelverkehrt angeordnet.

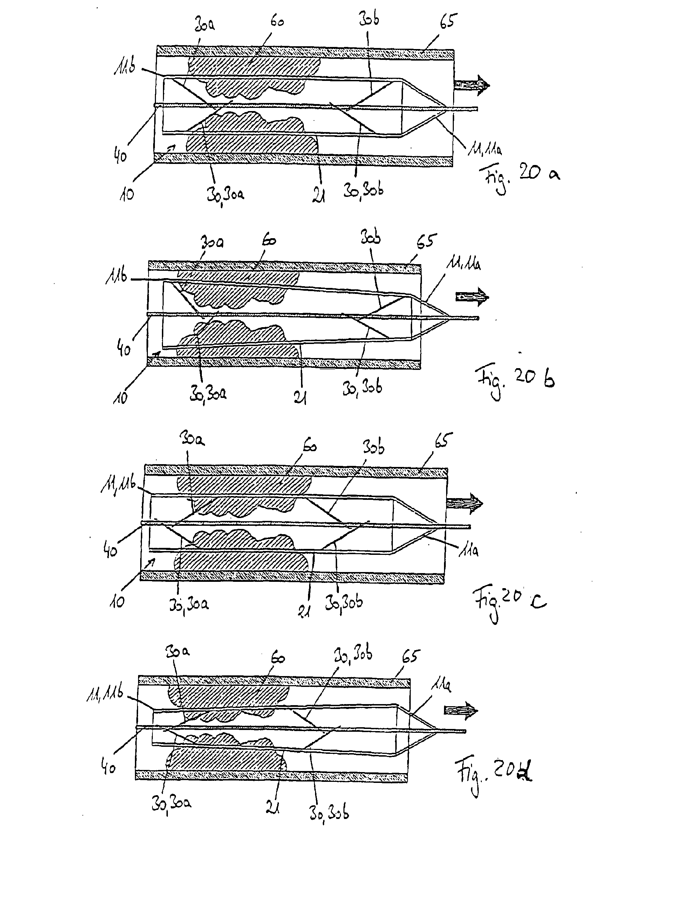

Die Fig. 20a bis 20d zeigen Ausführungsbeispiele der medizinischen Vorrichtung, wobei das Funktionselement 10 mehrere Halteelemente 30 aufweist, die unter schiedliche Richtungsorientierungen umfassen. Bei dem Ausführungsbeispiel gemäß Fig. 20a sind beispielweise zwei erste Halteelemente 30a vorgesehen, die in axialer Richtung versetzt angeordnet sind und dieselbe Richtungsorientierung aufweisen.

Zwei zweite Halteelemente 30b sind ebenfalls axial versetzt zueinander angeordnet und weisen dieselbe Richtungsorientierung auf. Die Richtungsorientierung der zweiten Halteelemente 30b ist gegenüber der Richtungsorientierung der ersten Halteelemente 30a umgekehrt. Das bedeutet, dass die Spitzen 31 der ersten Halteelemente 30a und der zweiten Halteelemente 30b zueinander gerichtet sind. Konkret sind die ersten Halteelemente 30a in distaler Richtung, und die zweiten Halteelemente 30b in proximaler Richtung ausgerichtet.

Durch eine geeignete Anordnung der Halteelemente 30, insbesondere durch geeignete Einstellung des axialen Versetzungsabstandes der Halteelemente 30 kann das Expansionsverhalten des Funktionselements 10 beeinflusst werden, wie in Fig. 20b dargestellt. Dabei sind die zweiten Halteelemente 30b derart axial zueinander versetzt angeordnet, dass bei einem vorgegebenen Expansionsgrad des Funktionselements 10 die Spitzen 31 der zweiten Halteelemente 30b aneinander anliegen und einer weiteren Expansion des Funktionselements entgegenwirken. Die Expansion des Funktionselements 10 wird im Bereich der zweiten Halteelemente 30b somit begrenzt. Auf diese Weise ergibt sich im expandierten Zustand des Funktionselements 10 eine im Wesentlichen kegelstumpfförmige Kontur der rotationssymmetrischen Gitterstruktur 20.

Bei dem Ausführungsbeispiel gemäß Fig. 20b sind die zweiten Halteelemente 30b am proximalen distalen Ende 11a des Funktionselements 10 angeordnet, so dass sich die Gitterstruktur 20 im expandierten Zustand des Funktionselements 10 in proximaler Richtung verjüngt. Alternativ kann der Axialabstand der ersten Halteelemente 30a zueinander eingestellt werden, so dass sich die Gitterstruktur 20 im expandierten Zustand des Funktionselements 10 in distaler Richtung verjüngt, wie in Fig. 20d dargestellt. Es ist im Übrigen möglich, dass sowohl der Axialabstand der ersten Halteelemente 30a, als auch der Axialabstand der zweiten Halteelemente 30b derart eingestellt ist, dass sich die Spitzen 31 der Halteelemente bei einem vorgegebenen Expansionsgrad des Funktionselements 10 berühren. Somit kann beispielsweise eine im expandierten Zustand des Funktionselements 10 zylinderförmige Gitterstruktur bereitgestellt werden, deren maximaler Expansionsdurchmesser begrenzt ist. Die Begrenzung des Expansionsdurchmessers sowohl über die gesamte Gitterstruktur 10, als auch in axialen Abschnitten der Gitterstruktur 10, hat den Vorteil, dass ein Kontakt der Gitterstruktur 20 bzw. des Funktionselements 10 mit der Gefäßwand eines Körperhohlorgans vermieden wird.

Das Ausführungsbeispiel gemäß Fig. 20c entspricht im Wesentlichen dem Ausführungsbeispiel gemäß Fig. 20a, wobei die Orientierung der ersten Halteelemente 30a und der zweiten Halteelemente 30b jeweils umgekehrt ist. Das bedeutet, dass die ersten Halteelemente 30a gemäß Fig. 20c in distaler Richtung und die zweiten Halteelemente 30b in proximaler Richtung orientiert sind. Dieselbe Orientierung weisen die Halteelemente 30a, 30b gemäß Fig. 20b auf.

In den Fig. 20a bis 20d ist überdies gut zu erkennen, dass der Schneidbereich 21, der durch die Stege 22 der Gitterstruktur 20 gebildet ist, im Gebrauch in das Konkrement 60 eindringt bzw. in das Konkrement 60 einschneidet. Die Halteelemente 30 werden hingegen aufgrund der Verbindung mit dem Führungsdraht 40 radial nach innen ausgelenkt und bilden somit eine Verankerung bzw. Fixierung für das Konkrement 60.

Die Begrenzung der Expansion des Funktionselements 10 kann vorteilhaft durch die Anordnung wenigstens eines Begrenzungselements 41 am Führungsdraht 40 unterstützt werden. In den Fig. 21a, 21b sind zwei Hülsen 41b dargestellt, die auf dem Führungsdraht 40 angeordnet sind und jeweils ein Begrenzungselement 41 bilden. Die Hülsen 41b sind mit dem Führungsdraht 40 fest verbunden. Die Ausrichtung und Anordnung der Halteelemente 30 bei dem Ausführungsbeispiel gemäß Fig. 21a, 21b entspricht im Wesentlichen der Ausrichtung und Anordnung der Halteelemente 30 gemäß dem Ausführungsbeispiel, das in den Fig. 21a, 21b dargestellt ist. Die Halteelemente 30 sind axial verschieblich mit dem Führungsdraht 40 verbunden. Das bedeutet, dass die Halteelemente 30 entlang des Führungsdrahts 40 gleiten können. Die Gleitbewegung wird durch die Hülsen 41b bzw. allgemein durch die Begrenzungselemente 41 begrenzt, wie in Fig. 21b dargestellt. Konkret zeigt Fig. 21a den teilexpandierten Zustand des Funktionselements 10, wobei die Halteelemente 30 frei auf dem Führungsdraht 40 gleiten können. In Fig. 21b ist der vollexpandierte Zustand des Funktionselements 10 dargestellt, wobei die Gleitbewegung jeweils eines ersten Halteelements 30a und eines zweiten Halteelements 30b durch die Hülsen 41b gestoppt ist. Die Hülse 41b bildet somit einen Anschlag für das Halteelement 30.

Alternativ oder zusätzlich zu der Hülse 41b kann eine Nut 41a als Begrenzungselement 41 vorgesehen sein, wie in den Fig. 22a, 22b dargestellt. Dabei zeigt Fig. 22a den teilexpandierten Zustand des Funktionselements 10, wobei die Halteelemente 30 frei auf dem Führungsdraht 40 gleiten können. Die Halteelemente 30 weisen eine Spitze 31 auf, die als Schlaufe 31b oder Öse 31a ausgebildet ist. Bei weitergehender Expansion des Funktionselements 10 gleitet jeweils ein erstes Halteelement 30a und ein zweites Halteelement 30b in jeweils eine Nut 41a. Die Öse 31a bzw. Schlaufe 31b der Spitze 31 des Halteelements 30 rastet somit in die Nut 41a ein (Fig. 22b). Die Expansion des Funktionselements 10 wird somit gestoppt.

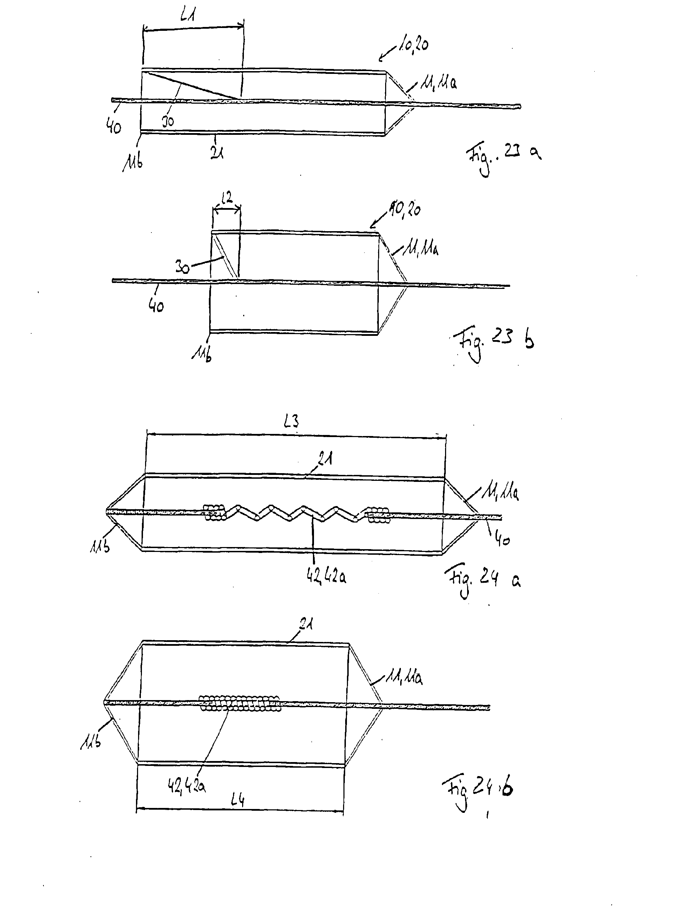

Beim Übergang vom radial komprimierten in den radial expandierten Zustand des Funktionselements wird nicht nur der Querschnittsdurchmesser der rotationssymmetrischen Gitterstruktur 20 erhöht, sondern gleichzeitig die axiale Länge der Gitterstruktur 20 verkürzt. Durch axial verschiebliche Verbindung der Halteelemente 30 mit dem Führungsdraht 40 können die Halteelemente 30 der Verkürzung der Gitterstruktur 20 bei der Expansion des Funktionselements 10 folgen. Es ist allerdings auch möglich, dass ein Halteelement 30 vorgesehen ist, das fest mit dem Führungsdraht 40 verbunden ist, wie in den Fig. 23a, 23b dargestellt. Dabei weist das Halteelement 30 vorzugsweise elastische Eigenschaften auf, die die Verkürzung der Gitterstruktur 20 bei der Expansion des Funktionselements 10 bzw. der Gitterstruktur 20 kompensieren. Konkret kann die Länge L1, L2 des Halteelements 30 bei der Expansion des Funktionselements 10 variieren. Insbesondere ist vorgesehen, dass das Halteelement 30, das fest mit dem Führungsdraht 40 verbunden ist, im komprimierten Zustand des Funktionselements 10 eine größere Länge L1, L2 aufweist (Fig. 23a) als im expandierten Zustand des Funktionselements 10 (Fig. 23b).

Um die Längenänderung der Gitterstruktur 20 bzw. des Funktionselements 10 bei der Expansion auszugleichen, kann ferner vorgesehen sein, dass der Führungsdraht 40 ein flexibles Element 42 umfasst. In den Fig. 24a, 24b ist eine derartige Konstruktion dargestellt, wobei das flexible Element 42 als Feder 42a ausgebildet ist. Aus Gründen der Übersichtlichkeit sind bei dem Ausführungsbeispiel gemäß Fig. 24a, 24b die Halteelemente 30, die mit dem Führungsdraht 40 fest verbunden sind, nicht gezeigt. Das Funktionselement 10 weist im komprimierten Zustand eine Länge L3 auf, die größer ist als die Länge L4 des Funktionselements 10 im expandierten Zustand. Im komprimierten Zustand des Funktionselements 10 ist die Feder 42a daher gestreckt. Die Feder 42a ist Teil des Führungsdrahts 40. Die Feder 42a bzw. allgemein das flexible Element 42 kann mit dem Führungsdraht 40 einteilig ausgebildet sein. Alternativ kann das flexible Element 42 bzw. die Feder 42a ein separates Bauteil bilden, das mit dem Führungsdraht 40 verschweißt, verklebt, gelötet oder durch Crimpen verbunden ist. Vorzugsweise ist das flexible Element 42 bzw. die Feder 42a derart angepasst, dass im komprimierten Zustand des Funktionselements eine Vorspannung bewirkt wird. Die in axialer Richtung des Funktionselements 10 wirkende Vorspannkraft kann die Expansion des Funktionselements 10 wirksam unterstützen.

Insbesondere kann die Feder 42a das Funktionselement 10 aktiv zusammenziehen, sobald das Funktionselement 10 aus dem Katheter 50 entlassen ist. Dabei erhöht sich gleichzeitig der Querschnittsdurchmesser des Funktionselements 10, so dass die Feder 42a aktiv zur Expansion des Funktionselements 10 beiträgt.

Für alle Ausführungsbeispiele gilt, dass die Gitterstruktur 20 im Allgemeinen, konkret die Stege 22, und/oder die Halteelemente 30 eine erhöhte Oberflächenrauhigkeit oder allgemein eine Oberflächenstrukturierung umfassen können. Damit wird eine biologisch aktive Oberfläche bereitgestellt, die eine biochemische Verbindung des Konkrements 60 bzw. eines Thrombus mit dem Funktionselement 10 begünstigt. Ferner können die Stege 22 bzw. allgemein die Gitterstruktur 20 mit Substanzen beschichtet sein, die eine klebende Wirkung aufweisen oder biologisch aktiv sind, um die Verbindung des Funktionselements 10 mit dem Konkrement 60 bzw. einem Thrombus zu verstärken. Beispielsweise können thrombogene Substanzen auf die Gitterstruktur 20 aufgebracht sein.

Um die mechanische Steifigkeit bzw. mechanischen Eigenschaften der Halteelemente 30 einzustellen, können an Stelle der Breite der Speichen 33a, 33b, bzw. Stege 22 auch Winkelverhältnisse zwischen den Stegen 22 und den Speichen 33a, 33b des Halteelements 30 eingestellt werden. Konkret können die Speichen 33a, 33b der Halteelemente 30 in Bezug auf die Längsachse des Funktionselements 10 einen anderen Winkel aufweisen, als die Stege 22 des Schneidbereichs 21 der Gitterstruktur 20. Beispielsweise können die Winkel der Speichen 33a, 33b des Halteelements 30 derart eingestellt sein, dass sich mehrere Halteelemente 30 bei der Expansion des Funktionselements 10 überlappen, so dass das Material des Konkrements 60 bzw. Thrombenmaterial zwischen zwei Halteelementen 30 praktisch eingequetscht wird.

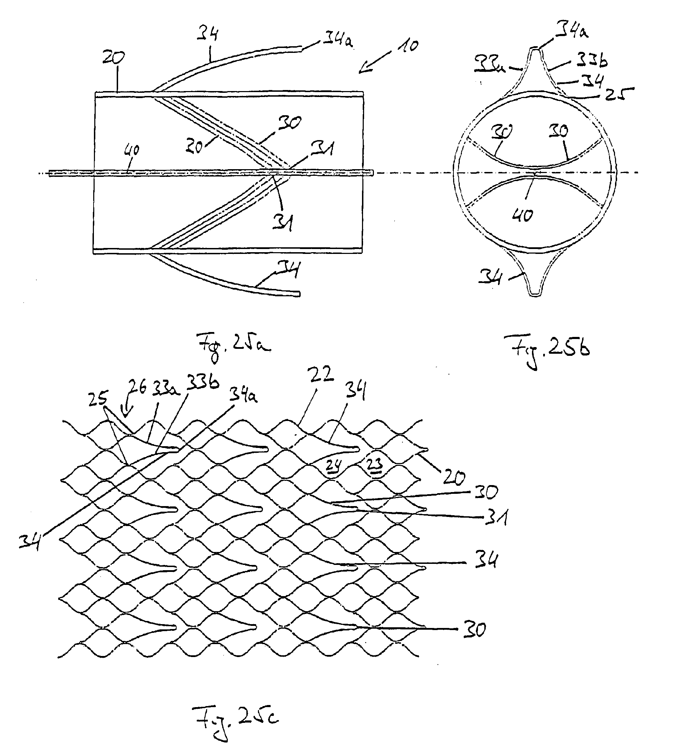

Bei den Ausführungsbeispielen gemäß den Fig. 25a bis 29 ist vorgesehen, dass die Gitterstruktur 20 wenigstens ein Fängerelement 34 umfasst, das im expandierten Zustand der Gitterstruktur 20 radial nach außen über die Wandung der Gitterstruktur 20 vorsteht. Durch das Fängerelement 34 wird die Fangwirkung des Funktionselements 10 verbessert. Wie vorstehend beschrieben, wird die Gitterstruktur 20 im Gebrauch nicht vollständig bis auf den Gefäßdurchmesser aufgedehnt. Vielmehr ist der Durchmesser des Funktionselements 10 kleiner als der Gefäßdurchmesser, so dass die Gitterstruktur 20 die Gefäßwand nicht berührt. Dadurch entsteht im Idealfall ein Ringspalt zwischen der Gefäßwand und der Gitterstruktur 20 des Funktionselements, der zumindest teilweise von dem zu entfernenden Konkrement 60 ausgefüllt ist. Da das Fängerelement 34 radial nach außen über die Gitterstruktur 20 vorsteht, deckt das Fängerelement 34 den Spalt zwischen der Gitterstruktur 20 und der Gefäßwand ab und nimmt beim Zurückziehen des Funktionselements 10 die im Spalt befindlichen Teile des Konkrements mit. Dadurch wird die Wirksamkeit des Funktionselements verbessert, da analog wie die nach innen gerichteten Halteelemente 30 der wirksame Querschnitt des Funktionselements 10 vergrößert wird und zwar radial nach außen. Dabei liegt nicht die komplette Gitterstruktur 20 an der Gefäßwand an, sondern nur das Fängerelement 34 bzw. mehrere einzelne Fängerelemente 34. Wie nachstehend im einzelnen ausgeführt, können die Fängerelemente 34 atraumatisch ausgebildet sein, so dass eine im Vergleich zum Stand der Technik schonendere Entfernung des Konkrements ermöglicht wird, selbst wenn einzelne Teile des Funktionselements 10 die Gefäßwand berühren.

Die Ausgestaltung des Funktionselements 34 wird anhand der nachfolgenden Ausführungsbeispiele näher erläutert. Wie in Fig. 25a zu erkennen, weist das Funktionselement 10 wenigstens ein, insbesondere mehrere Fängerelemente 34 auf, die im expandierten Zustand des Funktionselements 20 radial nach außen über die Gitterstruktur 20 vorstehen. Es kann ausreichend sein, ein einziges Fängerelement 34 vorzusehen. Zweckmäßigerweise sind mehreren Fängerelemente 34 auf dem Umfang der Gitterstruktur 20 angeordnet, beispielsweise zwei Fängerelemente 34 auf derselben Querschnittshöhe. Die Fängerelemente 34 können diametral entgegengesetzt auf dem Umfang der Gitterstruktur 20 angeordnet sein, wie in Fig. 25b beispielhaft dargestellt. Es ist auch möglich, mehr als 2, beispielsweise 3, 4, 5 oder mehr Fängerelemente 34 auf dem Umfang verteilt auf derselben Querschnittshöhe vorzusehen. Eine andere Anzahl von Fängerelementen auf derselben Querschnittshöhe ist ebenfalls möglich. Ferner können mehrere Fängerelemente 34 in Längsrichtung des Funktionselements voneinander beabstandet angeordnet sein. Diese Anordnung kann mit der Anordnung der auf dem Umfang verteilt angeordneten Fängerelemente 34 kombiniert werden. Die Fängerelemente 34 können in Längsrichtung fluchtend angeordnet sein. Es ist auch möglich, die Fängerelemente 34 in Längsrichtung voneinander beabstandet und in Umfangsrichtung zueinander versetzt anzuordnen. Dabei kann beispielsweise eine spiralförmige Verteilung der Fängerelemente 34 über den Umfang vorgesehen sein. Andere Verteilungsmuster der Fängerelemente 34 sind möglich.

Die Fängerelemente 34 können mit den Halteelementen 30, die sich in das Lumen des Funktionselements erstrecken, kombiniert werden. Dazu werden die Merkmale aller Ausführungsbeispiel einschließlich der in den Ansprüchen genannten Merkmale mit den Merkmalen der Ausführungsbeispiele zusammen offenbart, die das Fängerelement 34 betreffen, insbesondere die Ausführungsbeispiele gemäß Fig. 25a bis 29. Ein Beispiel für die Kombination des Fängerelements 34 bzw. der Fängerelemente 34 mit den Halteelementen 30 ist in den Fig. 25a bis 25c dargestellt. Beispielsweise können zwei Halteelemente 30 mit zwei Fängerelementen 34 kombiniert werden, die jeweils auf derselben Querschnittshöhe angeordnet sind. Diese Anordnung kann in Längsrichtung des Funktionselements wiederholt werden, wie in Fig. 25c verdeutlich. Die Anordnung der Fängerelemente 34 und der Halteelemente 30 ist auf derselben Querschnittshöhe um 90 Grad versetzt (Fig. 25b). In der Abwicklung gemäß Fig. 25c sind die Fängerelemente 34 und die Halteelemente 30 in Umfangsrichtung abwechselnd angeordnet, woraus sich bei der zylindrischen Form der Gitterstruktur 90 die in Fig. 25b gezeigte Anordnung ergibt. Die Verbindung der Fängerelemente 34 und der Halteelemente 30 erfolgt pro Ringsegment bzw. pro Zellenring 26 auf derselben Höhe, wie in den Fig. 25a und 25c zu erkennen. Eine beabstandete Verbindung der Fängerelemente 34 und der Halteelemente 30 sowie Kombinationen der unterschiedlichen Verbindungsstellen sind möglich.

Die Form der Fängerelemente 34 entspricht der Form der Halteelemente 30. Der Unterschied zwischen den Fängerelementen 34 und den Halteelementen 30 besteht darin, dass die Fängerelemente 34 im expandierten Zustand nach außen über die Wandung der Gitterstruktur 20 vorstehen, wohingegen die Halteelemente 30 in das Lumen des Funktionselements hineinragen, also radial nach innen über die Wandung der Gitterstruktur 20 vorstehen. Dabei ist das von der Gitterstruktur 20 beabstandete Ende bzw. die Spitze 31 des Halteelements 30 bzw. einiger Halteelemente 30 mit dem Führungsdraht 40 verbunden, wie in den Fig. 25a, 25b dargestellt. Das von der Gitterstruktur 20 beabstandete Ende der Fängerelemente ist ein freies Ende 34a. Das freie Ende 34a ist im expandierten Zustand außerhalb der Gitterstruktur 20 angeordnet, so dass zwischen dem freien Ende 34a und der Gitterstruktur 20 ein Abstand besteht.

Wie in Fig. 25c dargestellt, entspricht die Form der Fängerelemente 34 der Form der Halteelemente 30. Konkret sind die Fängerelemente 34 V-förmig ausgestaltet. Die Fängerelemente 34 weisen wie die Halteelemente 30 Speichen 33a, 33b auf, die zum freien Ende 34a bzw. der Spitze der Fängerelemente 34 hin zusammenlaufen und dort verbunden sind. Zur Gitterstruktur 20 hin sind die Speichen 33a, 33b verbreitert. Die dem freien Ende 34a entgegengesetzten Enden des Fängerelements 34 sind mit der Gitterstruktur 20 verbunden, insbesondere im Bereich von Knotenpunkten 25 der Gitterstruktur 20.

Die vorstehend beschriebene Form der Fängerelemente 34 ist mit Ausnahme des frei angeordneten Endes 34a ebenfalls bei den Halteelementen 30 vorgesehen. Es ist auch möglich, die Fängerelemente 34 und die Halteelemente 30 im Hinblick auf die Form unterschiedlich auszubilden.

Das Fängerelement 34 ist mit seinem freien Ende 34a in distaler Richtung ausgerichtet. Dies bedeutet, dass das freie Ende 34a zum distalen Ende des Funktionselements 10, also vom Katheter weg orientiert ist. Die Öffnung des V-förmigen Fängerelements 34 weist in proximale Richtung, d.h. zum Katheter hin. Der Bezug auf den Katheter zur Definition der Orientierung des Fängerelements 34 betrifft den entlassenen Zustand des Funktionselements, bei dem das Funktionselement 10 distal von der Katheterspitze angeordnet ist. Die Öffnung des Fängerelements 34 in proximaler Richtung ist einerseits für die Fangwirkung und andererseits für die traumatische Bewegung des Fängerelements 34 wichtig. Bei dem Ausführungsbeispiel gemäß Fig. 25a bis 25c sind die Fängerelemente 34 und die Halteelemente 30 in derselben Richtung ausgerichtet. Es ist auch möglich, die Fängerelemente 34 und die Halteelemente 30 in unterschiedlichen Richtungen auszurichten derart, dass die Fängerelemente 34 mit ihrem freien Ende 34a in distaler Richtung und die Halteelemente 30 mit dem mit dem Führungsdraht 40 verbundenen Ende bzw. der Spitze 31 in proximaler Richtung orientiert sind. Es ist auch möglich, mit dem freien Ende 34a in distaler Richtung orientierte Fängerelemente 34 mit Halteelementen 30 zu kombinieren, die in unterschiedliche Richtungen, d.h. in proximale Richtung und in distale Richtung orientiert sind. Hierzu wird Bezug genommen auf die vorstehend in Zusammenhang mit den Halteelementen erläuterten Ausführungsbeispiele.

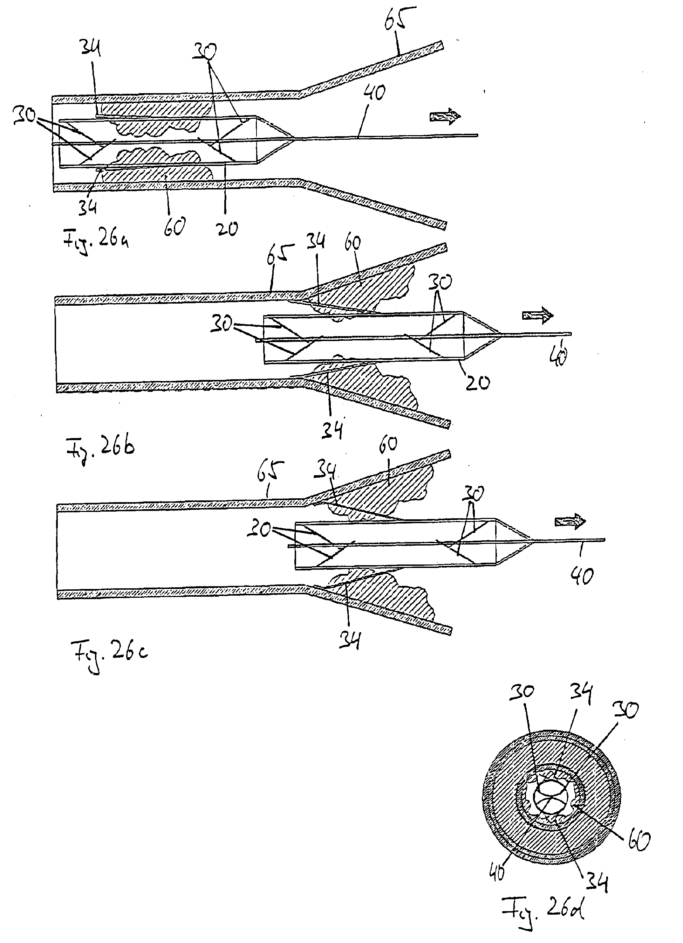

Die Anwendung eines Funktionselements 10 mit Fängerelement 34 ist in den Fig. 26a bis 26c dargestellt. Das Funktionselement 10 gemäß Fig. 26a umfasst zwei diametral auf dem Umfang angeordnete Fängerelemente 34 (entsprechend Fig. 25b). Die Halteelemente 30 sind in unterschiedlichen Querschnittshöhen angeordnet derart, dass die Fängerelemente 34 in Längsrichtung zwischen den Halteelementen 30 mit der Gitterstruktur 20 verbunden sind. Diese mögliche Anordnung der Fängerelemente 34 und der Halteelemente 30 auf unterschiedlichen Querschnittshöhen, wird allgemein und im Zusammenhang mit allen übrigen Ausführungsbeispielen offenbart. Die Halteelemente 30 sind in X-Anordnung vorgesehen. Die Spitzen 31 der Halteelemente 30 sind dabei jeweils in Längsrichtung nach innen orientiert. Der Führungsdraht 40 ist mit dem proximalen Ende des Funktionselements 10 fest verbunden. Der Katheter ist in den Fig. 26a bis 26c nicht dargestellt. Die Rückzugsrichtung ist durch einen Pfeil angedeutet. In den Fig. 26a bis 26c ist ferner dargestellt, dass sich der Gefäßdurchmesser in proximaler Richtung vergrößert.

Bei dem Behandlungsschritt gemäß Fig. 26a ist das Funktionselement 10 im Bereich des Konkrements 60 expandiert. Der Aufdehndurchmesser des Funktionselements 10 ist kleiner als der Gefäßdurchmesser, so dass sich zwischen der Gitterstruktur 20 und der Gefäßwand ein Spalt bildet. Der Schneidbereich der Gitterstruktur 20 dringt radial in das Konkrement 60 ein, wie in Fig. 26a zu erkennen. Durch die Einwirkung des Konkrements wird das Fängerelements 34 in dem in Fig. 26a gezeigten Zustand unmittelbar nach der Entlassung des Funktionselements 10 an die Gitterstruktur 20 gedrückt. Wird das Funktionselement 10 in Pfeilrichtung bewegt (Fig. 26b), wird das Konkrement 60 aufgrund der Verankerung des Schneidbereichs im Konkrement 60 mitgenommen. Die sich in das Lumen des Funktionselements 10 erstreckenden Halteelemente dienen hierbei zur Sicherheit für den Fall, dass der Schneidbereich der Gitterstruktur 20 nicht ausreichend im Konkrement 60 verankert ist.

In Fig. 26b ist zu erkennen, dass mit zunehmendem Gefäßdurchmesser das Konkrement 60 und die Gitterstruktur 20 bzw. deren Schneidbereich außer Eingriff kommen. Die Wirksamkeit des Schneidbereichs des Funktionselements 10 nimmt daher ab. Die Fängerelemente 34 folgen dem zurückweichenden Konkrement 60, so dass die Verankerung zwischen dem Konkrement 60 und dem Funktionselement 10 selbst dann gegeben ist, wenn die Gitterstruktur 20 bzw. der Schneidbereich der Gitterstruktur 20 und das Konkrement 60 vollständig außer Eingriff sind (Fig. 26c). Dann übernehmen die Fängerelemente 34 die Haltefunktion, da das Konkrement durch die Rückzugsbewegung in die V-förmig angeordneten Speichen 33a, 33b des Fängerelements 34 gedrängt und dort aufgrund der V-Form des Fängerelements 34 zusammengequetscht werden. Aufgrund der radial über die Gitterstruktur 20 nach außen vorstehenden Fängerelemente folgen diese zumindest bis zu einem bestimmten Grad der sich erweiternden Gefäßwand und stellen dadurch sicher, dass die Verbindung zwischen dem Konkrement 60 und dem Funktionselement 10 erhalten bleibt. Die Verbindung zwischen dem Konkrement 60 und den Fängerelementen 34 kann ausreichen, um das Konkrement 60 vollständig aus dem Gefäß zu entfernen.

Das Eindringen der Fängerelemente 34 in das Konkrement ist in Fig. 26d im Querschnitt gut zu erkennen.

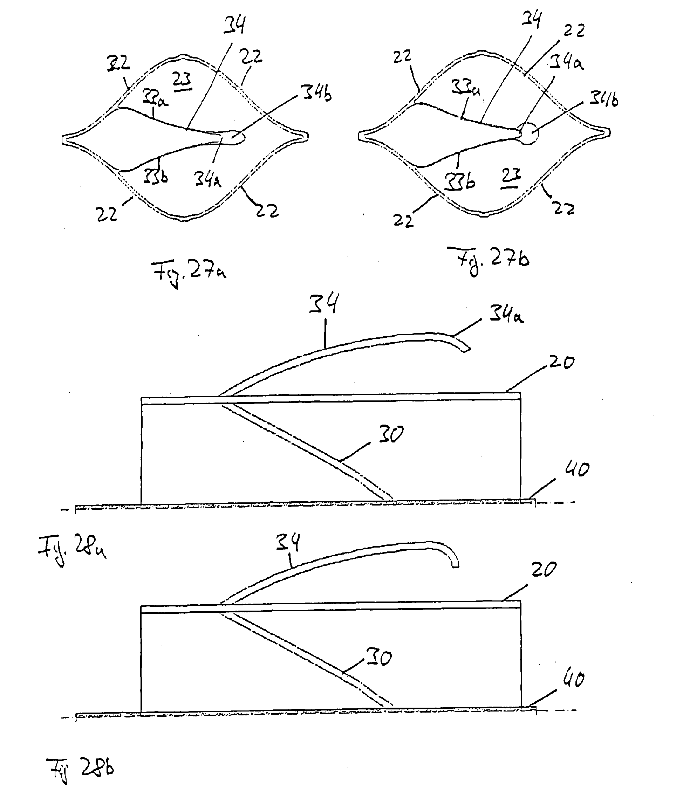

Zur Verbesserung der elastischen Eigenschaften der Fängerelemente 34 mit dem Ziel, die Traumatisierung des Gefäßes möglichst zu minimieren, ist vorgesehen, dass die Fängerelemente 34 möglichst lang sind und möglichst viele Stege bzw. Speichen 33a, 33b aufweisen. Konkret beträgt die Länge der Speichen 33b, 34b des Fängerelements 34 wenigstens die Hälfte, insbesondere wenigstens zwei Drittel, insbesondere wenigstens drei Viertel der Länge einer Zelle 23 in Längsrichtung des Funktionselements 10, in der das Fängerelement 34 angeordnet ist. Die vorstehend genannten Merkmale werden auch in Zusammenhang mit den Halteelementen 30 offenbart. Zum Schutz der Gefäßwand weisen die Fängerelemente 34 im Bereich des freien Endes 34a Stegverbreiterungen 34b auf, um die Krafteinleitung in die Gefäßwand großflächiger zu verteilen und somit schonender zu gestalten. Die Verbreiterung 34b kann beispielsweise länglich, insbesondere blattförmig sein (Fig. 27a). Die Verbreiterung 34b kann auch kreisförmig, insbesondere löffelartig sein (Fig. 27b). Andere Formen der Verbreiterung 34b sind möglich. Die Verbreiterung 34b schließt sich in Längsrichtung an das freie Ende 34a an und liegt im Wesentlichen auf der Mittelachse des Fängerelements 34 zwischen den V-förmige angeordneten Speichen 33a, 33b.

Es ist ferner möglich, dass das freie Ende 34a des Fängerelements 34 nach innen gebogen bzw. nach innen gekrümmt ist, derart, dass das freie Ende 34a in Richtung der Gitterstruktur 20 im expandierten Zustand weist (Fig. 28a, 28b). Das freie Ende 34a kann unterschiedlich stark in Richtung der Gitterstruktur 20 gekrümmt sein, wie in den Fig. 28a, 28b verdeutlicht. Damit wird erreicht, dass eine quer zur Einzugsrichtung verlaufende Außenkante des freien Endes 34a von der Gefäßwand sicher beabstandet ist und die Gefäßwand bei einer Bewegung des Fängerelements nur im Bereich der Krümmung relativ zum Fängerelement 34 gleitet.

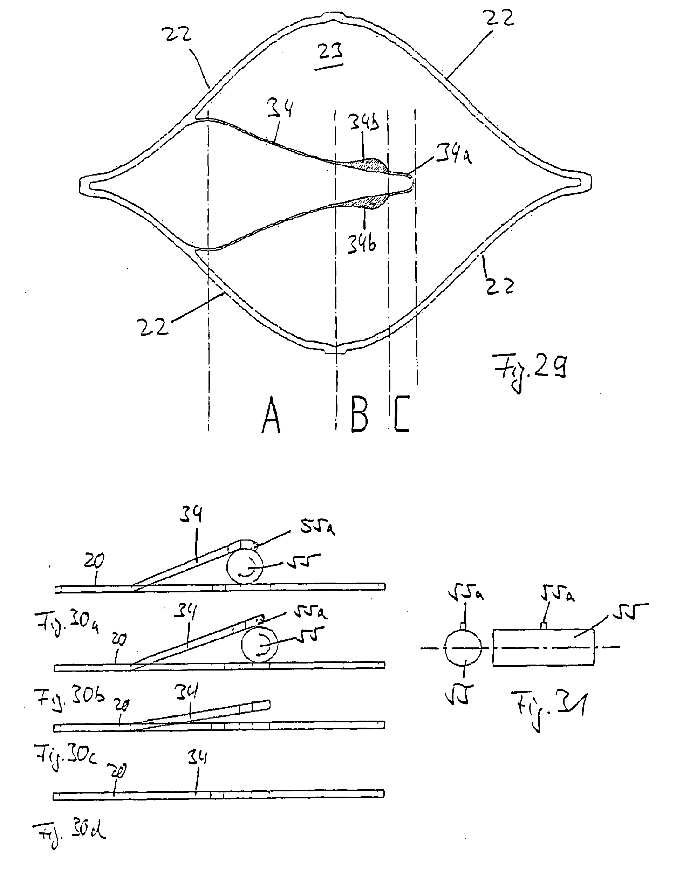

In Fig. 29 ist eine Kombination verschiedener Maßnahmen vorgestellt, die zu einer Verringerung der Traumatisierung der Gefäßwand beitragen. Die einzelnen Merkmale des Ausführungsbeispiels gemäß Fig. 29 werden sowohl in Kombination miteinander als auch jeweils für sich genommen einzeln offenbart und beansprucht. Wie in Fig. 29 ersichtlich, ist das Fängerelement 34 in drei Bereiche A, B, C unterteilt. Der proximale Bereich A zeichnet sich dadurch aus, dass die Speichen 33a, 33b bzw. Stege des Fängerelements 34 relativ dünn sind, insbesondere dünner als die Stege 22, die die Zelle 23 der Gitterstruktur 20 bilden. Dadurch wird die Flexibilität des Fängerelements 10 verbessert, so dass sich dieses gut an die Gefäßwand anlegen kann, ohne diese zu verletzen. Der mittlere Bereich B weist jeweils eine Stegverbreiterung 34b auf, die an den gegenüber angeordneten Speichen 33a, 33b vorgesehen sind. Der mittlere Bereich B ist distal zum Bereich A angeordnet. Der mittlere Bereich B ist nahe am freien Ende 34a des Fängerelements 34 angeordnet derart, dass die Verbreiterung 34b der beiden Speichen 33a, 33b bei der sukzessive zunehmenden Vergrößerung des Gefäßdurchmessers mit der Gefäßwand in Berührung kommt. Dadurch wird die vom Fängerelement 34 auf die Gefäßwand ausgeübte Kraft relativ großflächig verteilt. Das freie Ende 34a bildet den distalen Bereich C und ist zur Verringerung der Traumatisierung durch eine geeignete Wärmebehandlung, die an anderer Stelle näher beschrieben wird, radial nach innen gekrümmt.

Die Anordnung der Fängerelemente 34 ist in den Zellen 23 und/oder in den Maschen 24 möglich, wobei die Fängerelemente 34 in den Maschen 24 aufgrund der größeren Gitteröffnung entsprechend länger ausgebildet sein können. Die in Zusammenhang mit den Zellen erwähnten Längenverhältnisses (mindestens zwei Drittel, drei Viertel) werden auch im Zusammenhang mit den größeren Maschen 24 offenbart. Es ist auch möglich, die Fängerelemente 34 in den Maschen 24 kürzer auszubilden, beispielsweise wenn das Funktionselement 10 in einem Gefäß mit relativ geringer Zunahme des Gefäßdurchmessers in proximaler Richtung angewendet wird.

Zur Fertigung des nach innen gekrümmten freien Endes 34a kann bei Verwendung eines Formgedächtniswerkstoffes, der die Eigenschaft hat, nach einer Formänderung im martensitischen Zustand durch Erwärmen in den Ausgangszustand seine ursprüngliche Form nahezu vollständig oder teilweise wieder anzunehmen, der Formgedächtniseffekt genutzt werden. Dabei ist das Ende 34a im expandierten Zustand gekrümmt und im komprimierten Zustand gerade. Dadurch wird Crimpbarkeit des Funktionselements 10 verbessert und der Durchmesser des Funktionselements 10 im gecrimpten Zustand verringert.

Zur Herstellung ist ein Dorn 55 mit einem radial vorstehenden Stift 55a vorgesehen (siehe Fig. 31), der eine im wesentlichen zylindrische Form hat. Der Stift 55a wird in das freie Ende 34a bzw. die Spitze des Fängerelements 34 eingehängt und im Uhrzeigersinn gedreht, so dass das freie Ende 34a in Richtung der Lumenmitte bzw. in Richtung der Gitterstruktur 20 gebogen wird (sieh Fig. 30a). Durch Arretierung des Dorns und anschließende Wärmebehandlung bleibt die Deformation permanent. In der Tieftemperaturphase kann das freie Ende 34a zurückverformt werden derart, dass das Fängerelement 34 insgesamt flach ist und in die Gitterstruktur 20 eingepasst werden kann (Fig. 30b, 30d). Im Gebrauch wird das Fängerelement 34 erwärmt, wobei das Bauteil seine ursprüngliche, radial nach außen vorstehend und im Bereich des freien Endes 34a gekrümmte Form wieder annimmt.

Der Vollständigkeit halber wird offenbart, dass Konfigurationen des Funktionselements 10 möglich sind, bei denen die Elemente, d.h. die Fänger- und Halteelemente 34, 30 nur nach innen oder nur nach außen bzw. sowohl nach innen und nach außen gebogen sind. Es wird also auch ein Funktionselement offenbart, das nur nach außen ausgelenkte Fängerelemente 34 aufweist.

Es ist ferner möglich, den Schneidbereich der Gitterstruktur 20 so auszubilden, dass das Halteelement 30, also das nach innen auslenkbare Element, in Längsrichtung proximal und distal sowie in Umfangsrichtung der Gitterstruktur von Gitterzellen oder Maschen des Schneidbereichs umgeben ist, wobei diese Gitterzellen bzw. Maschen in derselben zylindrischen Mantelfläche der Gitterstruktur 20 angeordnet sind. Das bedeutet, dass ein Halteelement in allen Richtungen von Gitterzellen des Schneidbereichs umgeben ist, wodurch erreicht wird, dass sich die Gitterstruktur in den Thrombus um das Halteelement herum einschneidet. Durch die radial nach außen gerichtete Relativbewegung zwischen dem Konkrement und der Gitterstruktur 20 wird das Halteelement 30 vom Konkrement 60 nach innen in Richtung der Lumenmitte bewegt bzw. ausgelenkt.

Dabei ist nicht ausgeschlossen, dass die ein Halteelement 30 umgebenden Zellen 23 bzw. Maschen 24 selbst wiederum Halteelemente 30 beinhalten, so dass diese Zellen 23 bzw. Maschen 24 eine Doppelfunktion, nämlich einerseits die Befestigung der Halteelemente 30 und andererseits die Wirkung als Schneidzellen bzw. Schneidmaschen erfüllen. Durch die Anordnung des Schneidbereichs in derselben Mantelfläche wird erreicht, dass eine gleichmäßige Schneidwirkung des Funktionselements in Längsrichtung und in Umfangsrichtung der Gitterstruktur 20 erfolgt. Die Schneidwirkung und damit verbunden die Verankerungswirkung der den Schneidbereich bildenden Zellen 23 bzw. Maschen 24 wird dadurch verbessert, dass die Zellen 23 bzw. Maschen 24 im Wesentlichen rautenförmig ausgebildet sind, so dass die Stege 22 der Zellen 23 bzw. der Maschen 24 schräg bezogen auf die Längsachse des Funktionselements angeordnet sind. Die Stege sind im Bereich vom mehr als 0 Grad und weniger als 90 Grad bezogen auf die Längsachse angeordnet.

Bei einem wie vorstehend ausgeführten Funktionselement mit entsprechendem Schneidbereich kann auf die Fixierung der Halteelemente 30 am Führungsdraht 40 verzichtet werden, da in diesem Fall die Bewegung bzw. Auslenkung der Halteelemente 30 nach innen durch den Widerstand des Konkrements 60 erfolgt. Die Fixierung mittels des Führungsdrahts 40 hat den Vorteil, dass die Auslenkung der Halteelemente 30 in jedem Fall, also auch unabhängig von der Form des jeweiligen Konkrements erfolgt, wodurch die Sicherheit der Vorrichtung erhöht wird. Es ist möglich, den vorstehend beschriebenen Schneidbereich mit den Ausführungsbeispielen zu kombinieren, bei denen die Halteelemente 30 mit dem Führungsdraht verbunden sind, insbesondere fest oder lösbar verbunden sind. Es ist auch möglich, bei einem Funktionselement 10 Halteelemente frei, d.h. ohne Verbindung zum Führungsdraht und Halteelemente in Verbindung mit dem Führungsdraht vorzusehen.

Das Funktionselement 10 wird sowohl zusammen mit einem Katheter in Form einer Anordnung umfassend ein Funktionselement und einen Katheter offenbart und beansprucht. Das Funktionselement wird auch für sich alleine genommen, d.h. ohne den Katheter offenbart und beansprucht.

Das Funktionselement 10 kann ferner ein Aufstellelement 36 umfassen. Vorzugsweise weist das Funktionselement 10 mehrere Aufstellelemente 36 auf. Das Aufstellelement 36 weist im Wesentlichen dieselbe Form wie das Fängerelement 34 bzw. das Halteelement 30 auf. Mit anderen Worten kann das Aufstellelement 36 eine V-förmige Struktur umfassen. Das Aufstellelement 36 kann also entsprechend zwei V-förmig angeordnete Speichen aufweisen, wobei die Speichen in einer Spitze zusammenlaufen. Das Aufstellelement 36 ist mit wenigstens zwei Stegen 22 der Gitterstruktur 20 verbunden.

Das Aufstellelement 36 unterscheidet sich von dem Fängerelement 34 insbesondere durch seine Funktionsweise. Bei dem Fängerelement 34 ist vorgesehen, dass eine radiale Auslenkung durch den Übergang vom komprimierten Zustand in den expandierten Zustand der Gitterstruktur 20 erfolgt. Ein derartiger Mechanismus ist bei dem Aufstellelement 36 nicht vorgesehen. Vielmehr erfolgt die radial nach außen gerichtete Auslenkung des Aufstellelements 36 ausschließlich durch eine Krümmung des Funktionselements 10 bzw. der Gitterstruktur 20 entlang der Längsachse. Dabei ist vorteilhaft vorgesehen, dass die Spitze des Aufstellelements 36 in distale Richtung weist.

Die besondere Funktionsweise der Aufstellelemente 36 kann durch die geometrischen Abmessungen der Aufstellelemente 36 eingestellt werden. Dazu ist vorteilhaft vorgesehen, dass die Aufstellelemente eine Speichenbreite S1 aufweisen, die höchstens 60 µm, insbesondere höchstens 50 µm, insbesondere höchstens 40 µm, insbesondere höchstens 30 µm, insbesondere höchstens 20 µm, beträgt. Das Aufstellelement 36 ragt vorzugsweise in eine Zelle 23 hinein, wobei die die Zelle 23 begrenzenden Stege 22 eine Stegbreite S2 aufweisen, die höchstens 70 µm, insbesondere höchstens 60 µm, insbesondere höchstens 50 µm, insbesondere höchstens 40 µm, insbesondere höchstens 30 µm beträgt. Im Allgemeinen weisen die Aufstellelemente 36 also eine Speichenbreite S1 auf, die kleiner oder gleich der Stegbreite S2 der Stege 22 ist, die das Aufstellelement 36 umgeben. Besonders bevorzugt ist ein Verhältnis zwischen der Speichenbreite S1 des Aufstellelements 36 und der Stegbreite S2 der umgebenden Stege 22 (S1/S2) von höchstens 1,0, insbesondere höchstens 0,9, insbesondere höchstens 0,8, insbesondere höchstens 0,7, insbesondere höchstens 0,6, insbesondere höchstens 0,5.

Die Funktionsweise des Aufstellelements 36 bzw. der Aufstellelemente 36 wird nachfolgend anhand der Fig. 32 bis 32c erläutert.

Das Funktionselement 10 wird zunächst im Blutgefäß 65 im Bereich des Konkrements 60 positioniert und expandiert. Dabei schneidet der Schneidbereich 21 der Gitterstruktur 20 in das Konkrement 60 ein, so dass sich eine Verbindung zwischen dem Konkrement 60 und dem Funktionselement 10 ergibt (Fig. 32a). Zum Entfernen des Konkrements 60 wird das Funktionselement 10 anschließend in proximale Richtung bewegt bzw. gezogen. Beim Passieren einer Gefäßkrümmung 66 wird das Funktionselement 10 gebogen bzw. gekrümmt. Insbesondere bewirkt das Passieren der Gefäßkrümmung 66 eine Längskrümmung des Funktionselements 10. Mit anderen Worten wird die Rotationsachse der Gitterstruktur 20 aus einer geradlinigen Anordnung in eine gekrümmte bzw. gebogene Anordnung überführt. Dadurch wird auf einer Außenseite der Längskrümmung, also auf einer Seite der Gitterstruktur 20, die einen größeren Krümmungsradius aufweist, als die gegenüberliegende Seite der Gitterstruktur 20, ein Hohlraum 67 freigelegt.

Der Hohlraum 67 bietet einen Ausweichraum für das Konkrement 60, so dass sich das Konkrement 60 von dem Fängerelement 10 lösen kann. Die sichere Entfernung des Konkrements 60 aus dem Blutgefäß 65 ist dadurch gefährdet.

Die Bildung des Hohlraums 67 wird insbesondere dadurch begünstigt, dass durch das Ziehen des Funktionselements 10 eine axiale Verlängerung der Gitterstruktur 20 im Bereich von Gefäßkrümmungen 66 verursacht wird. Die axiale Verlängerung bzw. Längung der Gitterstruktur 20 bewirkt ferner eine radiale Kompression des Funktionselements 10 bzw. der Gitterstruktur 20. Das Funktionselement 10 füllt somit nicht mehr den gesamten Querschnittsdurchmesser des Blutgefäßes 65 aus. Vielmehr wird in einem Außenbereich der Gefäßkrümmung 66 der Hohlraum 67 gebildet.

Der gebildete Hohlraum 67 bietet Platz für das Konkrement 60, so dass sich das Konkrement 60 von dem Funktionselement 10 lösen kann. Dies wird durch die Aufstellelemente 36 verhindert. Durch die Längskrümmung der Gitterstruktur 20, die durch Ziehen des Funktionselements 10 durch die Gefäßkrümmung 66 bewirkt ist, stellen sich die Aufstellelemente 36 selbsttätig radial nach außen auf. Dabei kann vorgesehen sein, dass sich die Aufstellelemente 36 unterschiedlich stark aus der Wandungsebene der Gitterstruktur 20 erheben bzw. radial nach außen aufstellen. Die Auslenkung der Aufstellelemente 36 kann vom lokalen Krümmungsradius der Gitterstruktur 20 abhängig sein. Mit anderen Worten können die Aufstellelemente 36 bezogen auf die Gitterstruktur 20 denselben Aufstellwinkel aufweisen, wobei durch abschnittsweise unterschiedliche Längskrümmungsradien der Gitterstruktur 20 der Abstand der Spitzen der Aufstellelemente 36 zur Gitterstruktur 20 variiert. Die Aufstellelemente 36 ragen dabei in den Hohlraum 67 hinein. Somit bilden die Aufstellelemente 36 im ausgelenkten Zustand eine Sperre bzw. eine Fixierung für das Konkrement 60 (Fig. 32b).

Die Aufstellelemente 36 können in das Konkrement 60 eingreifen bzw. sich in das Konkrement 60 einhaken, so dass das Konkrement 60 weiterhin am Funktionselement 10 fixiert ist. Durch weiteres proximales Ziehen des Funktionselements 10 wird das Konkrement 60 auf diese Weise durch die Gefäßkrümmung 66 geführt (Fig. 32c). Somit bewirken die durch die anatomischen Bedingungen im Blutgefäß 65 aktivierten Aufstellelemente 36 ein sicheres Entfernen des Konkrements 60. Insbesondere wird durch die Aufstellelemente 36 die Sicherheit gegen einen Verlust des zu entfernenden Konkrements 60 erhöht.

Die Aufstellelemente 36 sind in distale Richtung ausgerichtet. Das bedeutet, dass die Aufstellelemente 36 Spitzen aufweisen, die in distale Richtung weisen. Auf diese Weise wird die Verletzungsgefahr für die Gefäßwand verringert. Vorzugsweise sind die Aufstellelemente 36 derart angepasst bzw. dimensioniert, dass sie in den Hohlraum 67 einer Gefäßkrümmung 66 hineinragen, ohne die Gefäßwand zu berühren.

In den Fig. 33 und 34 sind bevorzugte Dimensionen des Aufstellelements 36 dargestellt. Insbesondere zeigen die Fig. 33 und 34 bevorzugte geometrische Verhältnisse der Steglängen bzw. Zelllängen und der Erstreckung des Aufstellelements 36. Ferner sind für die Funktion der Aufstellelemente 36 die Abmessungen der Zellen 23 von Bedeutung, in denen das Aufstellelement 36 angeordnet ist bzw. die Abmessungen der Zellen 23, die keine Aufstellelemente 36 aufweisen.

Die Gesamtlänge der Zelle 23, in der kein Aufstellelement 36 angeordnet ist, wird als Gesamtlänge L8 der Zelle 23 ohne Aufstellelement 36 bezeichnet. Die Gesamtlänge der Zelle 23, in der ein Aufstellelement 36 angeordnet ist, wird als Gesamtlänge L9 der Zelle 23 mit einem Aufstellelement 36 bezeichnet. Wie aus Fig. 33 hervorgeht, beträgt die Gesamtlänge L9 der Zelle 23 mit einem Aufstellelement 36 ein Vielfaches der Gesamtlänge L8 der Zelle 23 ohne ein Aufstellelement 36.

Grundsätzlich sind die Zellen 23 durch Stege 22 begrenzt. Dabei unterteilt das Aufstellelement 36 zwei Stege 22 der Zelle 23 in axiale Abschnitte. Konkret ist das Aufstellelement 36 mit einem Steg 22 der Zelle 23 verbunden, wobei ein Knotenpunkt 25 gebildet ist. Ferner sind die Stege 22 benachbarter Zellen 23 miteinander in Knotenpunkten 25 verbunden. Der Knotenpunkt 25 in dem das Aufstellelement 36 mit dem Steg 22 gekoppelt ist, wird auch als Ansatzpunkt 25a bezeichnet. Der axiale Abstand zwischen dem Ansatzpunkt 25a und dem nächsten Knotenpunkt 25, der der Spitze des Aufstellelements 36 gegenüber angeordnet ist, wird als axialer Ansatzabstand L10 bezeichnet. Dabei wird der axiale Ansatzabstand L10 entlang der Längsachse des Funktionselements 10 bestimmt.

Die Bestimmung des axialen Ansatzabstands L10 erfolgt grundsätzlich im expandierten Zustand der Gitterstruktur 10. Der axiale Ansatzabstand L10 unterscheidet sich daher von einer Ansatzlänge L5, die zwischen dem nächsten Knotenpunkt 25, der der Spitze des Aufstellelements 36 gegenüber angeordnet ist, und dem Ansatzpunkt 25a entlang des Stegs 22 gemessen ist (Fig. 34).

Das Aufstellelement 36 weist ferner eine axiale Länge L11 auf, die der axialen Erstreckung zwischen den Ansatzpunkt 25a und der Spitze des Aufstellelements 36 entspricht. Die axiale Länge L11 des Aufstellelements 36 wird ebenfalls im expandierten Zustand der Gitterstruktur entlang der Längsachse der Gitterstruktur 20 ermittelt. Die axiale Länge L11 des Aufstellelements 36 unterscheidet sich somit von einer Speichenlänge L7 des Aufstellelements 36. Die Speichenlänge L7 des Aufstellelements 36 entspricht dem unmittelbaren Abstand zwischen dem Ansatzpunkt 25a und der Spitze des Aufstellelements 36, wie in Fig. 37 dargestellt. Mit anderen Worten entspricht die Speichenlänge L7 der Länge einer Speiche des Aufstellelements 36, wobei der geradlinige Abstand zwischen einem distalen Ende der Speiche, das in die Spitze übergeht, und einem proximalen Ende der Speiche, das im Wesentlichen dem Ansatzpunkt 25a entspricht, zugrunde gelegt wird. Die Messung erfolgt also nicht entlang der abschnittsweise gekrümmten Speiche, sondern entlang einer gedachten Linie bzw. Strecke zwischen den Enden der Speiche. Die Speichenlänge L7 des Aufstellelements 36 entspricht somit der kürzesten Verbindung zwischen dem Ansatzpunkt 25a und dem distalen Ende der Speiche bzw. der Spitze des Aufstellelements 36. Ebenso wie alle anderen relevanten Maße wird auch die Speichenlänge L7 des Aufstellelements 36 im expandierten Zustand der Gitterstruktur 20 ermittelt.

Der Steg 22, der den Ansatzpunkt 25a umfasst, weist einerseits die Ansatzlänge L5 auf, die dem Abstand zwischen einem Knotenpunkt 25 und dem Ansatzpunkt 25a, gemessen entlang des Stegs 22, entspricht. Ferner weist der Steg 22 eine Reststeglänge L6 auf. Die Reststeglänge L6 entspricht dem kürzesten Abstand zwischen dem Ansatzpunkt 25a und einem Knotenpunkt 25, der in Umfangsrichtung der Gitterstruktur 20 benachbart zum Aufstellelement 36 angeordnet ist. Die Ansatzlänge L5 und die Reststeglänge L6 sind in Fig. 34 anschaulich dargestellt.

Die Gitterstruktur 20 weist bezüglich der Aufstellelemente 36 vorzugsweise folgende Dimensionsverhältnisse auf:
Das Verhältnis zwischen der Gesamtlänge L9 der Zelle 23 mit einem Aufstellelement 36 zur Gesamtlänge L8 der Zelle 23 ohne ein Aufstellelement 36 (L9/L8) beträgt vorzugsweise höchstens 3,1, insbesondere höchstens 3,0, insbesondere höchstens 2,9, insbesondere höchstens 2,5, insbesondere höchstens 2,4, insbesondere höchstens 2,0, insbesondere höchstens 1,9, insbesondere höchstens 1,5. Das Verhältnis der axialen Länge L11 des Aufstellelements 36 zum axialen Ansatzabstand L10 (L11/L10) beträgt vorzugsweise höchstens 3,3, insbesondere höchstens 3,0, insbesondere höchstens 2,5, insbesondere höchstens 2,4, insbesondere höchstens 1,9, insbesondere höchstens 1,5, insbesondere höchstens 1,0. Das Verhältnis zwischen der Gesamtlänge L8 der Zelle 23 ohne ein Aufstellelement 36 und dem axialen Ansatzabstand L10 (L8/L10) beträgt vorzugsweise höchstens 2,5, insbesondere höchstens 2,3, insbesondere höchstens 2,0, insbesondere höchstens 1,8, insbesondere höchstens 1,5. Vorzugsweise weist die Gitterstruktur 10 ein Dimensionsverhältnis zwischen der Gesamtlänge L8 der Zelle 23 ohne ein Aufstellelement 36 und der axialen Länge L11 des Aufstellelements 36 (L8/L11) auf, das höchstens 2,0, insbesondere höchstens 1,8, insbesondere höchstens 1,5, insbesondere höchstens 1,2, insbesondere höchstens 1,0, insbesondere höchstens 0,8, insbesondere höchstens 0,5, beträgt. Entsprechende Dimensionsverhältnisse sind in Fig. 33 schematisch dargestellt.

Die Längenverhältnisse zwischen Ansatzlänge L11, Reststeglänge L6 und Speichenlänge L7 des Aufstellelements 36 sind in Fig. 34 schematisch dargestellt. Dabei ist vorteilhafterweise vorgesehen, dass das Verhältnis zwischen der Ansatzlänge L5 und der Reststeglänge L6 (L5/L6) höchstens 2,0, insbesondere höchstens 1,8, insbesondere höchstens 1,5, insbesondere höchstens 2,2, insbesondere höchstens 1,0, insbesondere höchstens 0,7, insbesondere höchstens 0,5, beträgt. Das Verhältnis zwischen der Speichenlänge L7 des Aufstellelements 36 und der Reststeglänge L6 (L7/L6) beträgt vorzugsweise höchstens 2,5, insbesondere höchstens 2,2, insbesondere höchstens 2,0, insbesondere höchstens 1,8, insbesondere höchstens 1,5, insbesondere höchstens 1,3, insbesondere höchstens 1,0.

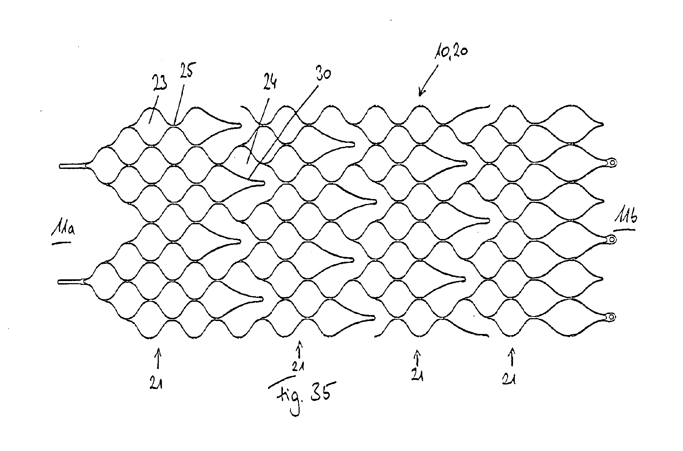

Fig. 35 zeigt ein bevorzugtes Design eines Funktionselement 10, der medizinischen Vorrichtung, wobei die Maschen 24 mit Halteelementen 30 in Umfangsreihen zickzack-förmig versetzt angeordnet sind. Zwischen zwei Umfangsseiten mit Maschen 24 sind jeweils Schneidbereiche 21 vorgesehen, die Zellen 23 ohne Halteelemente 30 umfassen. Die Halteelemente 30 sind in distale Richtung ausgerichtet.

In Fig. 35 sind überdies zwei paddels dargestellt (kein Bezugszeichen), die eine bevorzugte Form zur Verbindung der Vorrichtung bzw. des Funktionselements 10 mit dem Transportdraht bilden. Die paddels können gegenüberliegend angeordnet sein. Die Schweißung des Transportdrahts an die paddels erfolgt an beiden Seiten in zwei separaten Schritten oder auch in einem einzigen Schweißschritt.

Die Herstellung des Funktionselements 10 erfolgt vorzugsweise durch Laserschneiden. Dabei wird ein zylindrisches Halbzeug, als Rohmaterial verwendet, in das die Gitter-struktur 20 mittels Laserschneiden eingebracht wird. Die Gitterstruktur 20 kann anschließend einer Wärmebehandlung unterzogen werden, so dass die nach innen gerichtete Auslenkung der Halteelemente 30 bereits bei der Herstellung festgelegt ist.

Das Rohmaterial kann beispielsweise eine Nickel-Titan-Legierung aufweisen oder allgemein ein pseudoelastisches Material, insbesondere ein Formgedächtnismaterial. Durch die Wärmebehandlung wird die nach innen ausgelenkte Ausrichtung der Halteelemente 30 dem Formgedächtnismaterial aufgeprägt, so dass sich die Halteelemente 30 bei der Expansion des Funktionselements 10 im Wesentlichen selbsttätig radial nach innen auslenken. Die wärmebehandelten Halteelemente können auch mit dem Führungsdraht 40 kombiniert werden. Durch die Wärmebehandlung bei der Herstellung kann dieser Effekt jedoch begünstigt werden. Zusätzlich ist die Wärmebehandlung vorteilhaft, wenn freie Halteelemente 30 vorgesehen sind, die nicht mit dem Führungsdraht 40 verbunden sind. Alternativ zu Formgedächtnismaterialien kann die Gitterstruktur 20 bzw. allgemein das Funktionselement 10 auch Edelstahl umfassen.

Die Erfindung eignet sich insbesondere zur Entfernung von Thromben aus Blutgefäßen insbesondere aus zerebralen Gefäßabschnitten.

Zusammengefasst beruht die erfindungsgemäße Vorrichtung auf dem Gedanken, die Gitterstruktur 20 einerseits mit einem Schneidbereich 21 zu versehen, der dazu angepasst ist, beim Expandieren der Gitterstruktur in das am Gefäß anhaftende Konkrement 60 einzudringen, insbesondere einzuschneiden. Dem Schneidbereich 21 ist ein Halteelement 30, insbesondere in der Form einer Zunge, bzw. einer zungenförmigen Zelle mit einer Spitze zugeordnet. Das Halteelement 30 unterstützt die Verankerungswirkung des Schneidbereichs 21. Dazu ist die Zunge flexibel mit der Gitterstruktur 20 verbunden derart, dass beim Eindringen des Schneidbereichs 21 in das Konkrement dieses gegen die Zunge drückt und die Zunge radial nach innen auslenkt. Durch die radial nach innen auslenkbare Zunge wird zusammen mit dem Schneidbereich, der die Zunge zumindest teilweise umgibt, die Verankerung und damit der Widerstand des Funktionselements beim Entfernen des Konkrements aus dem Gefäß verbessert. Das Halteelement 30 bzw. die Zunge kann zusätzlich oder alternativ so ausgeführt sein, dass diese sich bei einer Längskrümmung des Funktionselements radial nach außen aus der zylindrischen Mantelfläche bewegt. Die Zunge kann damit dem Konkrement folgen, wenn sich der Schneidbereich, beispielsweise bei der Bewegung des Funktionselements entlang einer Gefäßkrümmung aus dem Konkrement löst. Dazu befindet sich die Zunge auf dem Außenradius des gekrümmten Funktionselements.

Im Ruhezustand bei expandiertem Funktionselement ist das Halteelement 30 bzw. die Zunge in derselben zylindrischen Mantelfläche wie der Schneidbereich angeordnet. Konkret ist die Spitze der Zunge im selben zylindrischen Mantelbereich bzw. derselben zylindrischen Mantelfläche der Gitterstruktur angeordnet.

Der vorstehend erläuterte Aufbau und die Funktionsweise der Vorrichtung wird im Zusammenhang mit allen Merkmalen der Beschreibung bzw. allgemein im Zusammenhang mit der Erfindung offenbart, ohne diese darauf einzuschränken.

### Bezugszeichenliste

- 10: Funktionselement
- 11: axiales Ende
- 11a: proximales Ende
- 11b: distales Ende
- 20: Gitterstruktur
- 21: Schneidbereich
- 22: Steg
- 22a: erster Steg
- 22b: zweiter Steg
- 23: Zelle
- 24: Masche
- 25: Knotenpunkt
- 25a: Ansatzpunkt
- 26: Zellenring
- 26a: erster Zellenring
- 26b: zweiter Zellenring
- 30: Halteelement
- 30a: erstes Halteelement
- 30b: zweites Halteelement
- 31: Spitze
- 31a: Öse
- 31b: Schlaufe
- 31c: Gleithülse
- 32: Kontaktfläche
- 33a: erste Speiche
- 33b: zweite Speiche
- 34: Fängerelement
- 34a: freies Ende
- 34b: Stegverbreiterung
- 35: Übergangsbereich
- 36: Aufstellelement
- 40: Führungsdraht
- 41: Begrenzungselement
- 41a: Nut
- 41b: Hülse
- 42: flexibles Element
- 42a: Feder
- 50: Katheter
- 55: Dorn
- 55a: Stift
- 60: Konkrement
- 65: Blutgefäß
- 66: Gefäßkrümmung
- 67: Hohlraum
- A: proximaler Endbereich
- B: distaler Endbereich
- L1: Länge des ersten Halteelements 30a
- L2: Länge des zweiten Halteelements 30b
- L3: Länge des Funktionselements 10 im komprimierten Zustand
- L4: Länge des Funktionselements 10 im expandierten Zustand
- L5: Ansatzlänge
- L6: Reststeglänge
- L7: Speichenlänge des Aufstellelements 36
- L8: Gesamtlänge der Zelle 23 ohne Aufstellelement 36
- L9: Gesamtlänge der Zelle 23 mit Aufstellelement 36
- L10: axialer Ansatzabstand
- L11: axiale Länge des Aufstellelements 36
- S1, S3: Speichenbreite
- S2: Stegbreite
- S4: Spitzenbreite
- S5: erste Stegbreite
- S6: zweite Stegbreite
- S7: Übergangsbreite

## Patentansprüche

1. Medizinische Vorrichtung zum Entfernen von Konkrementen (60) aus Körperhohlorganen mit einem Funktionselement (10), das eine rotationssymmetrische Gitterstruktur (20) und ein Mittel zum Halten eines Konkrements (60) in der Gitterstruktur (20) aufweist, und einem Katheter (50) zum Zuführen und Entfernen des Funktionselements (10) aus dem Körper, wobei das Funktionselement (10) von einem komprimierten Zustand im Katheter (50) in einen expandierten Zustand außerhalb des Katheters (50) überführbar ist, in welchem das Funktionselement (10) distal vom Katheter (50) angeordnet ist,
**dadurch gekennzeichnet, dass**
die Gitterstruktur (20) einen Schneidbereich (21) mit Stegen (22) aufweist, die im Gebrauch das Konkrement (60) bei der Überführung des Funktionselements (10) vom komprimierten Zustand in den expandierten Zustand zumindest teilweise radial durchdringen, und das Mittel zum Halten wenigstens ein Halteelement (30) umfasst, das in Längsrichtung proximal und distal sowie in Umfangsrichtung der Gitterstruktur (20) von Gitterzellen (23) und/oder Maschen (24) des Schneidbereichs umgeben ist, wobei die Gitterzellen (23) und/oder Maschen (24) des Schneidbereichs in derselben zylindrischen Mantelfläche der Gitterstruktur (20) angeordnet sind.

2. Vorrichtung nach Anspruch 1,
**dadurch gekennzeichnet, dass**
das Halteelement (30) eine zungenförmige Zelle mit einer freien Spitze bildet, die in einer rautenförmigen Gitterzelle und/oder Masche der Gitterstruktur (20) angeordnet ist.

3. Vorrichtung nach Anspruch 1 oder 2,
**dadurch gekennzeichnet, dass**
die Gitterzelle oder Masche, in der das Halteelement (30) angeordnet ist, flexibler als die Gitterzellen (23) und/oder Maschen (24) des Schneidbereichs ist, der das Halteelement (30) zumindest bereichsweise, insbesondere vollständig umgibt.

4. Vorrichtung nach einem der vorhergehenden Ansprüche,
**dadurch gekennzeichne**t, dass
die Gitterzelle oder Masche, in der das Halteelement (30) angeordnet ist, kleiner, gleich groß oder größer als die umgebenden Gitterzellen (23) oder Maschen (24) des Schneidbereichs sind.

5. Vorrichtung nach einem der vorhergehenden Ansprüche,
**dadurch gekennzeichnet, dass**
das Verhältnis der Steglänge der Gitterzelle oder Masche, in der das Halteelement (30) angeordnet ist, zur Steglänge der umgebenden Gitterzellen (23) oder Maschen (24) des Schneidbereichs mindestens 110%, insbesondere mindestens 120%, mindestens 130%, mindestens 140%, mindestens 150%, mindestens 175%, mindestens 200% beträgt.

6. Vorrichtung nach einem der vorhergehenden Ansprüche,
**dadurch gekennzeichnet, dass**
das Halteelement (30) Stege aufweist, deren Breite höchstens 150%, insbesondere höchstens 120%, höchstens 110%, höchstens 90%, höchstens 80%, höchstens 70%, höchstens 60%, höchstens 50%, höchstens 40% der Stegbreite der umgebenden Gitterzellen (23) oder Maschen (24) des Schneidbereichs aufweisen.

7. Vorrichtung nach einem der vorhergehenden Ansprüche,
**dadurch gekennzeichnet, dass**
das Verhältnis der Breite der Stege einer Gitterzelle oder Masche, in der das Halteelement (30) angeordnet ist, zur Stegbreite der umgebenden Gitterzellen (23) oder Maschen (24) höchstens 200%, insbesondere höchstens 150%, höchstens 120%, höchstens 90%, höchstens 80%, höchstens 70%, höchstens 60%, höchstens 50% aufweist.

8. Vorrichtung nach einem der vorhergehenden Ansprüche,
**dadurch gekennzeichnet, dass**
das Halteelement (30) im Wesentlichen V-förmig ausgebildet ist und zwei Speichen (33a, 33b) umfasst, die mit jeweils wenigstens einem Steg (22) des Schneidbereichs (21) verbunden sind.

9. Vorrichtung nach Anspruch 8,
**dadurch gekennzeichnet, dass**
die Speichen (33a, 33b) des Halteelements (30) eine kleinere mechanische Steifigkeit, insbesondere eine kleinere Breite und/oder Dicke, als die Stege (22) des Schneidbereichs (21) aufweisen.

10. Vorrichtung nach einem der vorhergehenden Ansprüche,
**dadurch gekennzeichnet, dass**
die Gitterstruktur (20) wenigstens ein Fängerelement (34) umfasst, das im expandierten Zustand radial nach außen über die Gitterstruktur (20) vorsteht.

11. Vorrichtung nach einem der vorhergehenden Ansprüche,
**dadurch gekennzeichnet, dass**
sich das Halteelement (30) im komprimierten Zustand des Funktionselements (10) in längsaxialer Richtung des Funktionselements (10) erstreckt.

12. Vorrichtung nach einem der vorhergehenden Ansprüche,
**dadurch gekennzeichnet, dass**
mehrere Halteelemente (30) vorgesehen sind, die unterschiedliche Längen aufweisen.

13. Vorrichtung nach einem der vorhergehenden Ansprüche,
**dadurch gekennzeichnet, dass**
in Längsrichtung der Gitterstruktur (20) wenigstens ein Ringsegment mit einem oder mit mehreren Halteelementen (30) und wenigstens ein Ringsegment ohne Halteelemente angeordnet ist.

14. Vorrichtung nach Anspruch 13,
**dadurch gekennzeichnet, dass**
das Verhältnis der Zellenanzahl eines Ringelements mit Halteelement (30) zur Zellenanzahl eines Ringelements ohne Halteelement höchstens 3/4, insbesondere höchstens 2/3, höchstens 1/2, höchstens 1/3 beträgt.

15. Vorrichtung nach Anspruch 13 oder 14,
**dadurch gekennzeichnet, dass**
das Verhältnis der Anzahl von Ringelementen mit wenigstens einem Halteelement (30) zur Anzahl von Ringelementen ohne Halteelement (30) mindestens 1:3, insbesondere mindestens 1:2, mindestens 2:3, mindestens 1:1, mindestens 3:2, mindestens 2:1, mindestens 3:1, mindestens 4:1 beträgt.

16. Medizinische Vorrichtung zum Entfernen von Konkrementen (60) aus Körperhohlorganen mit einem Funktionselement (10), das eine rotationssymmetrische Gitterstruktur (20) und ein Mittel zum Halten eines Konkrements in der Gitterstruktur (20) aufweist, und einem Katheter (50) zum Zuführen und Entfernen des Funktionselements (10) aus dem Körper, wobei das Funktionselement (10) von einem komprimierten Zustand im Katheter (50) in einen expandierten Zustand außerhalb des Katheters (50) überführbar ist, in welchem das Funktionselement (10) distal vom Katheter (50) angeordnet ist,
**dadurch gekennzeichnet, dass**
die Gitterstruktur (20) einen Schneidbereich (21) mit Stegen (22) aufweist, die angepasst sind derart, dass diese das Konkrement (60) bei der Überführung des Funktionselements (10) vom komprimierten Zustand in den expandierten Zustand zumindest teilweise radial durchdringen, und das Haltemittel wenigstens ein Halteelement (30) umfasst, das einerseits mit der Gitterstruktur (20) und andererseits mit einem im Katheter (50) angeordneten Führungsdraht (40) verbunden ist derart, dass das Halteelement (30) im expandierten Zustand des Funktionselements (10) radial nach innen ausgelenkt ist.

## Claims

1. Medical device for detaching concretions (60) from hollow organs of the body, having a functional element (10) which has a rotationally symmetrical lattice structure (20) and a means for holding a concretion (60) in the lattice structure (20), and having a catheter (50) for introduction and removal of the functional element (10) from the body, the functional element (10) being arranged to be changed from a compressed state inside the catheter (50) to an expanded state outside the catheter (50) wherein the functional element (10) is arranged distally from the catheter (50),
**characterised in that**
the lattice structure (20) has a cutting region (21) having webs (22) which, in use, radially penetrate, at least in part, the concretion (60) when the functional element (10) is changed from the compressed state to the expanded state; and the holding means comprises at least one holding element (30) which, in the longitudinal direction proximally and distally and also in the circumferential direction of the lattice structure (20), is surrounded by lattice cells (23) and/or meshes (24) of the cutting region, the lattice cells (23) and/or meshes (24) of the cutting region being arranged in the same cylindrical envelope surface of the lattice structure (20).

2. Device according to claim 1,
**characterised in that**
the holding element (30) forms a tongue-shaped cell having a free tip arranged in a lozenge-shaped lattice cell and/or mesh of the lattice structure (20).

3. Device according to claim 1 or 2,
**characterised in that**
the lattice cell or mesh in which the holding element (30) is arranged is more flexible than the lattice cells (23) and/or meshes (24) of the cutting region which surrounds the holding element (30) at least regionally, especially completely.

4. Device according to one of the preceding claims,
**characterised in that**
the lattice cell or mesh in which the holding element (30) is arranged is smaller than, the same size as or larger than the surrounding lattice cells (23) or meshes (24) of the cutting region.

5. Device according to one of the preceding claims,
**characterised in that**
the ratio of the web length of the lattice cell or mesh in which the holding element (30) is arranged to the web length of the surrounding lattice cells (23) or meshes (24) of the cutting region is at least 110 %, especially at least 120 %, at least 130 %, at least 140 %, at least 150 %, at least 175 %, at least 200 %.

6. Device according to one of the preceding claims,
**characterised in that**
the holding element (30) has webs whose width is at most 150 %, especially at most 120 %, at most 110 %, at most 90 %, at most 80 %, at most 70 %, at most 60 %, at most 50 %, at most 40 % of the web width of the surrounding lattice cells (23) or meshes (24) of the cutting region.

7. Device according to one of the preceding claims,
**characterised in that**
the ratio of the width of the webs of a lattice cell or mesh in which the holding element (30) is arranged to the web width of the surrounding lattice cells (23) or meshes (24) is at most 200 %, especially at most 150 %, at most 120 %, at most 90 %, at most 80 %, at most 70 %, at most 60 %, at most 50 %.

8. Device according to one of the preceding claims,
**characterised in that**
the holding element (30) is of substantially V-shaped construction and comprises two spokes (33a, 33b) each of which is connected to at least one web (22) of the cutting region (21).

9. Device according to claim 8,
**characterised in that**
the spokes (33a, 33b) of the holding element (30) have a lower mechanical rigidity, especially a smaller width and/or thickness, than the webs (22) of the cutting region (21).

10. Device according to one of the preceding claims,
**characterised in that**
the lattice structure (20) has at least one catching element (34) which, in the expanded state, projects radially outwards beyond the lattice structure (20).

11. Device according to one of the preceding claims,
**characterised in that**
the holding element (30), in the compressed state of the functional element (10), extends in the longitudinal axial direction of the functional element (10).

12. Device according to one of the preceding claims,
**characterised in that**
a plurality of holding elements (30) which have different lengths are provided.

13. Device according to one of the preceding claims,
**characterised in that**
in the longitudinal direction of the lattice structure (20) there is arranged at least one ring segment having one or a plurality of holding elements (30) and at least one ring segment without holding elements.

14. Device according to claim 13,
**characterised in that**
the ratio of the number of cells of a ring element having a holding element (30) to the number of cells of a ring element without a holding element is at most 3/4, especially at most 2/3, at most 1/2, at most 1/3.

15. Device according to claim 13 or 14,
**characterised in that**
the ratio of the number of ring elements having at least one holding element (30) to the number or ring elements without a holding element (30) is at least 1:3, especially at least 1:2, at least 2:3, at least 1:1, at least 3:2, at least 2:1, at least 3:1, at least 4:1.

16. Medical device for detaching concretions (60) from hollow organs of the body, having a functional element (10) which has a rotationally symmetrical lattice structure (20) and a means for holding a concretion in the lattice structure (20), and having a catheter (50) for introduction and removal of the functional element (10) from the body, the functional element (10) being arranged to be changed from a compressed state inside the catheter (50) to an expanded state outside the catheter (50) wherein the functional element (10) is arranged distally from the catheter (50),
**characterised in that**
the lattice structure (20) has a cutting region (21) having webs (22) which are so adapted that they radially penetrate, at least in part, the concretion (60) when the functional element (10) is changed from the compressed state to the expanded state; and the holding means comprises at least one holding element (30) which is so connected, on the one hand, to the lattice structure (20) and, on the other hand, to a guide wire (40) arranged in the catheter (50) that the holding element (30) is, in the expanded state of the functional element (10), deflected radially inwards.

## Revendications

1. Dispositif médical pour l'élimination de calculs (60) d'organes corporels creux comportant un élément fonctionnel (10) qui présente une structure en treillis symétrique en rotation (20) et un moyen de retenue d'un calcul (60) dans la structure en treillis (20) et un cathéter (50) pour l'acheminement et le retrait de l'élément fonctionnel (10) du corps, dans lequel l'élément fonctionnel (10) peut passer d'un état comprimé dans le cathéter (50) à un état déployé à l'extérieur du cathéter (50), dans lequel l'élément fonctionnel (10) est disposé sur le plan distal du cathéter (50),
**caractérisé en ce que**
la structure en treillis (20) présente une zone de coupe (21) dotée d'entretoises (22) qui pénètrent lors de l'utilisation dans le calcul (60) lors du passage de l'élément fonctionnel (10) de l'état comprimé à l'état déployé au moins partiellement au niveau radial et le moyen de retenue comprend au moins un élément de retenue (30) qui est entouré dans la direction longitudinale au niveau proximal et distal et dans le sens périphérique de la structure en treillis (20), par des cellules de treillis (23) et/ou des mailles (24) de la zone de coupe, dans lequel les cellules de treillis (23) et/ou les mailles (24) de la zone de coupe sont disposées sur la même surface de revêtement cylindrique de la structure en treillis (20).

2. Dispositif selon la revendication 1,
**caractérisé en ce que**
l'élément de retenue (30) forme une cellule en forme de langue dotée d'une pointe libre qui est disposée dans une cellule de treillis et/ou une maille de la structure en treillis (20) ayant une forme de losange.

3. Dispositif selon la revendication 1 ou 2,
**caractérisé en ce que**
la cellule en treillis ou la maille dans laquelle l'élément de retenue (30) est disposé, est plus souple que les cellules de treillis (23) et/ou les mailles (24) de la zone de coupe qui entoure l'élément de retenue (30) au moins partiellement, en particulier, complètement.

4. Dispositif selon l'une des revendications précédentes,
**caractérisé en ce que**
la cellule de treillis ou la maille dans laquelle l'élément de retenue (30) est disposé, est plus petite, de même taille ou plus grande que les cellules de treillis (23) ou les mailles (24) environnantes de la zone de coupe.

5. Dispositif selon l'une des revendications précédentes,
**caractérisé en ce que**
le rapport des longueurs d'entretoise de la cellule de treillis ou de maille dans laquelle l'élément de retenue (30) est disposé par rapport à la longueur d'entretoise des cellules de treillis (23) ou des mailles (24) de la zone de coupe environnantes, est d'au moins 110 %, en particulier, d'au moins 120 %, d'au moins 130 %, d'au moins 140 %, d'au moins 150 %, d'au moins 175 %, d'au moins 200 %.

6. Dispositif selon l'une des revendications précédentes,
**caractérisé en ce que**
l'élément de retenue (30) présente des entretoises dont la largeur est au plus de 150 %, en particulier au plus de 120 %, au plus de 110 %, au plus de 90 %, au plus de 80 %, au plus de 70 %, au plus de 60 %, au plus de 50 %, au plus de 40 % de la largeur d'entretoise des cellules de treillis (23) ou des mailles (24) de la zone de coupe environnantes.

7. Dispositif selon l'une des revendications précédentes,
**caractérisé en ce que**
le rapport de la largeur des entretoises d'une cellule de treillis ou de maille dans laquelle l'élément de retenue (30) est disposé, par rapport à la largeur d'entretoise des cellules de treillis (23) ou des mailles (24) environnantes est au plus de 200 %, en particulier au plus de 150 %, au plus de 120 %, au plus de 90 %, au plus de 80 %, au plus de 70 %, au plus de 60 %, au plus de 50 %.

8. Dispositif selon l'une des revendications précédentes,
**caractérisé en ce que**
l'élément de retenue (30) est essentiellement en forme de V et comprend deux rayons (33a, 33b) qui sont reliés avec respectivement une entretoise (22) de la zone de coupe (21).

9. Dispositif selon la revendication 8,
**caractérisé en ce que**
les rayons (33a, 33b) de l'élément de retenue (30) présentent une rigidité mécanique inférieure, en particulier, une largeur et/ou une épaisseur inférieure à celle des entretoises (22) de la zone de coupe (21).

10. Dispositif selon l'une des revendications précédentes,
**caractérisé en ce que**
la structure en treillis (20) comprend au moins un élément de capture (34) qui dépasse radialement vers l'extérieur à l'état déployé hors de la structure en treillis (20).

11. Dispositif selon l'une des revendications précédentes,
**caractérisé en ce que**
l'élément de retenue (30) s'étend à l'état comprimé de l'élément fonctionnel (10) dans la direction longitudinale de l'élément fonctionnel (10).

12. Dispositif selon l'une des revendications précédentes,
**caractérisé en ce que**
plusieurs éléments de retenue (30) sont prévus, qui présentent des longueurs différentes.

13. Dispositif selon l'une des revendications précédentes,
**caractérisé en ce que**
dans la direction longitudinale de la structure en treillis (20) est disposé au moins un segment annulaire comportant un ou plusieurs éléments de retenue (30) et au moins un segment annulaire sans élément de retenue.

14. Dispositif selon la revendication 13
**caractérisé en ce que**
le rapport du nombre de cellules d'un élément annulaire avec un élément de retenue (30) au nombre de cellules d'un élément annulaire sans élément de retenue est au plus de 3/4, en particulier au plus de 2/3, au plus de 1/2, au plus d'1/3.

15. Dispositif selon la revendication 13 ou 14,
**caractérisé en ce que**
le rapport du nombre d'éléments annulaires avec au moins un élément de retenue (30) au nombre d'éléments annulaires sans élément de retenue (30) est d'au moins 1 : 3, en particulier d'au moins 1 : 2, d'au moins 2: 3, d'au moins 1 : 1, d'au moins 3 : 2, d'au moins 2 : 1, d'au moins 3 : 1, d'au moins 4 : 1.

16. Dispositif médical pour l'élimination de calculs (60) d'organes corporels creux comportant un élément fonctionnel (10) qui présente une structure en treillis symétrique en rotation (20) et un moyen de retenue d'un calcul dans la structure en treillis (20) et un cathéter (50) pour l'acheminement et le retrait de l'élément fonctionnel (10) du corps, dans lequel l'élément fonctionnel (10) peut passer d'un état comprimé dans le cathéter (50) à un état déployé à l'extérieur du cathéter (50), dans lequel l'élément fonctionnel (10) est disposé sur le plan distal du cathéter (50),
**caractérisé en ce que**
la structure en treillis (20) présente une zone de coupe (21) dotée d'entretoises (22) qui sont adaptées de telle sorte que celles-ci pénètrent dans le calcul (60) lors du passage de l'élément fonctionnel (10) de l'état comprimé à l'état déployé au moins partiellement au niveau radial et le moyen de retenue comprend au moins un élément de retenue (30) qui est relié d'une part à la structure en treillis (20) et d'autre part à un fil guide (40) disposé dans le cathéter (50) de telle sorte que l'élément de retenue (30) soit orienté radialement vers l'intérieur à l'état déployé de l'élément fonctionnel (10).
